# FASCICULE DE BREVET EUROPEEN

(11) **EP 0 591 059 B1**
(45) Date de publication et mention de la délivrance du brevet: **15.04.1998**
(21) Numéro de dépôt: 93402396.1
(22) Date de dépôt: 01.10.1993
(51) Int. Cl.: A61K 7/13, C07C 323/26, C07C 323/36

(54) **Utilisation en teinture des fibres kératiniques de métaphénylènediamines soufrées et nouvelles métaphénylènediamines soufrées**
Verwendung von Schwefelenthaltenden Metaphenylenediaminen zur Färbung von Keratinfasern und neue Schwefelenthaltende Metaphenylene-Diamine
Use of sulfur-containing metaphenylenediamines for dyeing keratinic fibers and new sulfur-containing metaphenylenediamines

(30) Priorité: 02.10.1992 FR 9211711
(43) Date de publication de la demande: 06.04.1994
(73) Titulaire: L'OREAL, 75008 Paris (FR)
(72) Inventeur: Junino, Alex, F-93190 Livry-Gargan (FR); Lagrange, Alain, F-77700 Coupvray (FR); Genet, Alain, F-93600 Aulnay-sous-Bois (FR)
(74) Mandataire: Casalonga, Axel

(56) Documents cités:
- EP-A- 0 253 593
- WO-A-92/22525
- DE-A- 3 343 642
- FR-A- 1 061 331
- FR-A- 2 687 308
- US-A- 4 973 760
- US-A- 4 982 002
- INT. SAMPE SYMP.EXHIB. (ISSEEG) vol. 33 , Mars 1988 , ANAHEIM,CALIF. USA pages 746 - 753 R.B.GOSNELL ET AL. 'An ortho-thioalkylated aromatic diamine as an improved liquid hardener for carbon fiber reinforced epoxy matrices'
- PHOSPHORUS AND SULFUR AND THE RELATED ELEMENTS , GORDON & BREACH SCIENCE PUBLISHERS *Th.S.CROFT : Fluoroalkylthio substituted aromatic derivatives* * page 133 - page 139 * *page 136, exemple 37*

## Description

La présente invention est relative à l'utilisation pour la teinture de fibres kératiniques et plus particulièrement des cheveux humains, de métaphénylènediamines soufrées, à des compositions tinctoriales contenant ces métaphénylènediamines soufrées, à un procédé de teinture mettant en oeuvre ces compositions, ainsi qu'à de nouvelles métaphénylènediamines soufrées et leur procédé de préparation.

Les documents EP-0 253 593, US-A-4 982 002 et INT. SAMPE SYMP., vol. 33, mars 1988, p.746-753 décrivent des hydrocarbylthio diamines aromatiques et plus particulièrement les 3,5-diméthylthio 2,6-diaminotoluène, 3,5-di-méthylthio 2,4-diaminotoluène et 2,4,6-tri-éthylthio 1,3-diaminobenzène. Le document PHOSPHORUS AND SULPHUR AND THE RELATED ELEMENTS, Th. S.CROFT, p.133-139 décrit quant à lui le N,N'-di(2,2,2-trifluoroéthyle)-4,6-bis(trifluorométhylthio)-1,3-diaminobenzène.

On a déjà utilisé des dérivés soufrés d'amines aromatiques, associés à des précurseurs de colorants d'oxydation, pour la teinture de fibres kératiniques.

Il est connu de teindre les fibres kératiniques et en particulier les cheveux humains, avec des compositions tinctoriales contenant des précurseurs de colorants d'oxydation et des coupleurs.

Les coupleurs, encore appelés modificateurs de coloration, permettent de faire varier les nuances obtenues avec les précurseurs de colorants d'oxydation.

Dans le domaine de la teinture des fibres kératiniques et en particulier des cheveux humains, on est à la recherche de coupleurs qui, associés à des précurseurs de colorants d'oxydation, permettent d'obtenir un large éventail de nuances, tout en conférant aux cheveux une coloration ayant une résistance satisfaisante à la lumière, au lavage, aux intempéries, à la transpiration et aux différents traitements que peuvent subir les cheveux.

La demanderesse vient de découvrir, ce qui fait l'objet de l'invention, que l'utilisation de certaines métaphénylènediamines soufrées à titre de coupleurs avec des précurseurs de colorants d'oxydation de type ortho et/ou para et un agent oxydant dans des compositions tinctoriales pour fibres kératiniques, permettaient d'obtenir après application sur les fibres kératinques et en particulier les cheveux humains, un large éventail de nuances de coloration présentant une résistance à la lumière, au lavage, aux intempéries, à la transpiration et aux différents traitements que peuvent subir les cheveux, particulièrement remarquable.

La présente invention a donc pour objet l'utilisation des métaphénylènediamines soufrées définies ci-après pour la teinture des fibres kératiniques et en particulier les cheveux humains.

Un autre objet de l'invention est constitué par des compositions tinctoriales d'oxydation, destinées à être utilisées pour la teinture des fibres kératiniques et en particulier des cheveux humains, contenant au moins un précurseur de colorant d'oxydation de type ortho et/ou para et au moins une métaphénylènediamine soufrée de formule (I) définie ci-après.

Un autre objet de l'invention porte sur le procédé de coloration des fibres kératiniques et en particulier des cheveux humains, mettant en oeuvre une telle composition mélangée à un agent oxydant.

L'invention a également pour objet de nouvelles métaphénylènediamines soufrées ainsi que leur procédé de préparation.

D'autres objets de l'invention apparaîtront à la lecture de la description et des exemples qui suivent.

La présente invention a donc pour objet l'utilisation pour la teinture de fibres kératiniques et en particulier les cheveux humains d'au moins, une métaphénylènediamine soufrée de formule générale : 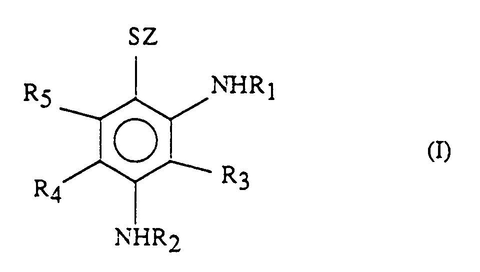 dans laquelle :
Z représente un radical alkyle en C₁-C₁₈, un radical aralkyle dans lequel le radical alkyle est en C₁-C₆, un radical monohydroxyalkyle en C₁-C₆ ou polyhydroxyalkyle C₂-C₆, un radical aryle, un radical fluoroalkyle en C₁-C₄,un radical aminoalkyle de formule : 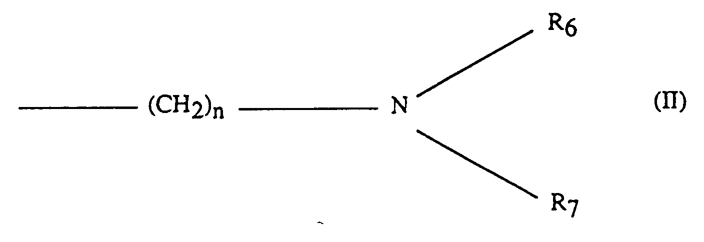 dans laquelle
   n est un nombre entier compris entre 1 et 6 inclus; R₆ et R₇, identiques ou différents, représentent un atome d'hydrogène ou un radical alkyle en C₁-C₄, hydroxyalkyle en C₁-C₄, acyle en C₁-C₆;
R₁ et R₂, identiques ou différents, représentent un atome d'hydrogène, un radical alkyle en C₁-C₆, un radical monohydroxyalkyle en C₁-C₆, polyhydroxyalkyle en C₂-C₆, monocarbamylalkyle en C₁-C₆, dialkyl-carbamyle en C₁-C₆, aminoalkyle en C₁-C₆, acylaminoalkyle (C₁-C₄), carbalcoxy (C₂-C₆) alkyle (C₁-C₄), carbamyle ou monoalkyl en C₁-C₆ carbamyle, fluoroalkyle en C₁-C₄;
R₃ représente un atome d'hydrogène, un radical alkyle en C₁-C₄;
R₄ et R₅, identiques ou différents, représentent un atome d'hydrogène, un radicale alkyle en C₁-C₄, un radical alcoxy en C₁-C₄, un radical hydroxyalkyle en C₁-C₄, un halogène, un radical -SZ où Z a les significations indiquées ci-dessus,
au moins un des radicaux R₃, R₄ ou R₅ est différent d'un atome d'hydrogène,
et leurs sels d'acides.

Parmi les significations préférées du radical Z dans les métaphénylènediamines soufrées de formule générale (I) selon l'invention, le radical alkyle en C₁-C₁₈ désigne les radicaux méthyle, éthyle, propyle, butyle, dodécyle, hexadécyle; le radical aralkyle désigne le radical benzyle ; le radical mono ou polyhydroxyalkyle désigne -CH₂-CH₂OH, -CH₂-CHOH-CH₂-OH, -CH₂-CHOH-CH₃;
le radical aryle désigne le phényle; le radical aminoalkyle désigne -CH₂-CH₂-NH₂, -CH₂-CH₂-NHCH₃,
le radical acylaminoalkyle désigne -CH₂-CH₂-NHCOCH₃ ; ou 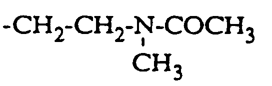 lorsque les groupements R₆ et R₇ représentent un radical acyle, celui-ci désigne de préférence les radicaux formyle, acétyle et propionyle.
Lorsque le groupement R₃ désigne un radical alkyle, celui-ci est un radical méthyle, éthyle, n-propyle, isopropyle, n-butyle ou isobutyle.

Les sels d'acides correspondants sont choisis de préférence parmi les chlorhydrates, les sulfates ou les bromhydrates.

Parmi les métaphénylènediarnines soufrées de formule générale (I), on peut citer :
le 2-méthyl-4-méthylthio-1,3-diaminobenzène dénommé selon la nomenclature IUPAC 2-méthyl-4-méthylsulfanyl-benzène-1,3-diamine,
le 2-éthyl-4-méthylthio-1,3-diaminobenzène dénommé selon la nomenclature IUPAC 2-éthyl-4-méthylsulfanyl-benzène- 1,3-diamine,
le 4-chloro-6-propylthio-1,3 diamino benzène dénommé selon la nomenclature IUPAC 4-chloro-6-propylsulfanyl-benzène-1,3-diamine,
le 4,6-bis-méthylthio-1,3-diaminobenzène dénommé selon la nomenclature IUPAC 4,6-bis-méthylsulfanyl-benzène-1,3-diamine
le 4,6-bis-éthylthio-1,3-diaminobenzène dénommé selon la nomenclature IUPAC le 4,6-bis-éthylsulfanyl-benzene-1,3-diamine
le 4,6-bis-trifluorométhylthio-1,3-[N,N'bis(2,2,2trifluoroéthyl)]-diamino benzène dénommé selon la nomenclature IUPAC N,N'-bis-(2,2,2-trifluoro-éthyl)-4,6-bis-trifluorométhylsulfanyl benzène-1,3-diamine
le 4-méthyl-6-méthylthio-1,3-diaminobenzène dénommé selon la nomenclature IUPAC 4-méthyl-6-méthylsulfanyl-benzène-1,3-diamine
le 4-éthylthio-6-méthyl-1,3-diamino benzène dénommé selon la nomenclature IUPAC 4-éthylsulfanyl-6-méthyl-benzène-1,3-diamine
le 4-carboxyéthylthio-6-carboxyméthylthio-1,3-diaminobenzène dénommé selon la nomenclature IUPAC acide 3-(2,4-diamino-5-carboxyméthylsulfanyl-phénylsulfanyl)-propionique
le 4,6-bis-carboxyéthylthio-1,3-diaminobenzène dénommé selon la nomenclature IUPAC acide 3-[2,4-diamino-5-(2-carboxyéthylsulfanyl)-phénylsulfanyl]-propionique
le 2-méthyl-4,6-bis-méthylthio-1,3-diaminobenzène dénommé selon la nomenclature IUPAC 2-méthyl-4,6-bis-méthylsulfanyl-benzène-1,3-diamine
le 4,6-bis-éthylthio-2-méthyl-1,3-diaminobenzène dénommé selon la nomenclature IUPAC 4,6-bis-éthylsulfanyl-2-méthyl-benzène-1,3-diamine
le 4,6-bis-propylthio-2-méthyl-1,3-diaminobenzène dénommé selon la nomenclature IUPAC 4,6-bis-propylsulfanyl-2-méthyl-benzène-1,3-diamine
le 4-méthoxy-6-β-acétylaminoéthylthio-1,3diaminobenzène dénommé selon la nomenclature IUPAC N-[2-(2,4-diamino-5-méthoxy-phénylsulfanyl)-éthyl]-acetamide
le 4-méthoxy-6-méthylthio-1,3-diaminobenzène dénommé selon la nomenclature IUPAC 4-méthoxy-6-méthylsulfanyl-benzène-1,3-diamine
le 5-chloro-2-méthyl-4-β-acétylaminoéthylthio-1,3-diaminobenzène dénommé selon la nomenclature IUPAC N[2-(2,4-diamino 6-chloro 3-méthylphényl sulfanyl)éthyl] acétamide.
le 4,6-bis-hydroxyéthylthio-1,3-diaminobenzène dénommé selon la nomenclature IUPAC 2-[2,4-diamino-5-(2-hydroxy-éthylsulfanyl)-phénylsulfanyl]-éthanol

Les composés de formule (I) sont utilisables comme coupleurs en présence de précurseurs de colorants d'oxydation de type ortho et/ou para connus en eux-mêmes, permettant de teindre les cheveux par coloration d'oxydation, selon un processus mettant en oeuvre une réaction de condensation oxydative des précurseurs et du coupleur.

Les précurseurs de colorants de type ortho et/ou para sont des composés qui ne sont pas des colorants en eux-mêmes, mais qui forment un colorant par un processus de condensation oxydative, soit sur eux-mêmes, soit en présence d'un coupleur ou modificateur.

Ces précurseurs de colorants d'oxydation de type ortho ou para sont des composés benzéniques ou hétérocycliques qui comportent deux groupements fonctionnels amino ou hydroxy et amino, en position ortho ou para l'un par rapport à l'autre.

Les précurseurs de colorants d'oxydation de type ortho ou para peuvent être choisis parmi les paraphénylènediamines, les paraaminophénols, les précurseurs hétérocycliques para dérivés de la pyridine, de la pyrimidine ou du pyrazole, tels que la 2,5-diaminopyridine, la 2-hydroxy 5-aminopyridine, la 2,4,5,6-tétraaminopyrimidine, le 4,5-diamino 1-méthylpyrazole, la 2-diméthylamino 4,5,6-triaminopyrimidine, les orthoaminophénols et les bases dites "doubles".

A titre de paraphénylènediamines, on peut plus particulièrement citer les composés répondant à la formule (III) : 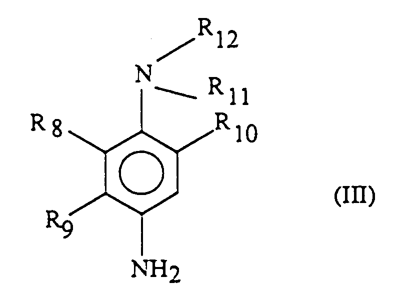 dans laquelle :
R₈, R₉, R₁₀, identiques ou différents, représentent un atome d'hydrogène ou d'halogène, un radical alkyle, un radical alcoxy, un radical carboxy, sulfo ou hydroxyalkyle en C₁-C₄ ;
R₁₁ et R₁₂, identiques ou différents, représentent un atome d'hydrogène, un radical alkyle, hydroxyalkyle, alcoxyalkyle, carbamylalkyle, mésylaminoalkyle, acétylaminoalkyle, uréidoalkyle, carbalcoxyaminoalkyle, sulfoalkyle, pipéridinoalkyle, morpholinoalkyle, ou phényle éventuellement substitué en para par un groupement amino.; ou bien R₁₁ et R₁₂ forment conjointement avec l'atome d'azote auquel ils sont liés, un hétérocycle pipéridino ou morpholino, sous réserve que R₈ ou R₁₀ représente un atome d'hydrogène lorsque R₁₁ et R₁₂ ne représentent pas un atome d'hydrogène, ainsi que les sels de ces composés. Ces radicaux alkyle ou alcoxy ont de préférence 1 à 4 atomes de carbone et désignent notamment les radicaux méthyle, éthyle, propyle, méthoxy et éthoxy.

Parmi les composés de formule (III) on peut plus particulièrement citer la paraphénylènediamine, la p-toluylènediamine, la méthoxyparaphénylènediamine, la chloroparaphénylènediamine, la 2,3-diméthylparaphénylènediamine, la 2,6-diméthylparaphénylènediamine, la 2,6-diéthylparaphénylènediamine, la 2,5-diméthylparaphénylènediamine, la 2-méthyl 5-méthoxyparaphénylènediamine, la 2,6-diméthyl 5-méthoxyparaphénylènediamine, la N,N-diméthylparaphénylènediamine, la N,N-diéthylparaphénylènediamine, la N,N-dipropylparaphénylènediamine, la 3-méthyl 4-amino N,N-diéthylaniline, la N,N-di-(β-hydroyyéthyl)paraphénylènediamine, la 3-méthyl 4-amino N,N-di-(β-hydroxyéthyl)aniline, la 3-chloro 4-amino N,N-di-(β-hydroxyéthyl)aniline, la 4-amino N,N-(éthyl, carbamylméthyl)-aniline, la 3-méthyl 4-amino N,N-(éthyl, carbamylméthyl)aniline, la 4-amino N,N-(éthyl, β-pipéridinoéthyl) aniline, la 3-méthyl 4-amino N,N-(éthyl, β-pipéridinoéthyl)aniline, la 4-amino N,N-(éthyl, β-morpholinoéthyl)aniline, la 3-méthyl 4-amino N,N-(éthyl,β-morpholinoéthyl)aniline, la 4-amino N,N-(éthyl,β-acétylaminoéthyl) aniline, la 4-amino N-(β-méthoxyéthyl) aniline, la 3-méthyl 4-amino N,N-(éthyl, β-acétylaminoéthyl) aniline, la 4-amino N,N-(éthyl, β-mésylaminoéthyl) aniline, la 3-méthyl 4-amino N,N-(éthyl, β-mésylaminoéthyl) aniline, la 4-amino N,N-(éthyl, β- sulfoéthyl)aniline, la 3-méthyl 4-amino N,N-(éthyl, β-sulfoéthyl) aniline, la N-[(4'-amino)phényl]-morpholine, la N-[(4'-amino)phényl]pipéridine, la 2-hydroxyéthylparaphénylènediamine, la fluoroparaphénylènediamine, la carboxyparaphénylènediamine, la sulfoparaphénylènediamine, la 2-isopropylparaphénylènediamine, la 2-n-propylparaphénylènediamine, l'hydroxy-2-n-propylparaphénylènediamine, la 2-hydroxyméthylparaphénylènediamine, la N,Ndiméthyl 3-méthyl paraphénylènediamine, la N,N-(éthyl,β-hydroxyéthyl)paraphénylènediamine, la N-(dihydroxypropyl)paraphénylènediamine, la N-4'-aminophénylparaphénylènediamine, la N-phénylparaphénylènediamine.

Ces paraphénylènediamines peuvent être utilisées soit sous forme de base libre, soit sous forme de sels, tels que chlorhydrate, bromhydrate ou sulfate.

Parmi les p-aminophénols, on peut citer le p-aminophénol, le 2-méthyl 4-aminophénol, le 3-méthyl 4-aminophénol, le 2-chloro 4-aminophénol, le 3-chloro 4-aminophénol, le 2,6-diméthyl 4-aminophénol, le 3,5-diméthyl 4-aminophénol, le 2,3-diméthyl 4-aminophénol, le 2,5-diméthyl 4-aminophénol, le 2-hydroxyméthyl 4-aminophénol, le 2-(β-hydroxyéthyl)4-aminophénol, le 2-méthoxy 4-aminophénol, le 3-méthoxy 4-aminophénol, le 3-(β-hydroxyéthoxy)4-aminophénol, le 2-méthoxyméthyl 4-aminophénol, le 2-aminométhyl 4-aminophénol, le 2-β-hydroxyéthylaminométhyl 4-aminophénol, le 2-éthoxyméthyl 4-aminophénol, le 2-(β-hydroxyéthoxy)méthyl 4-aminophénol.

Les bases dites "doubles" sont des bis-phénylalkylènediamines, répondant à la formule : 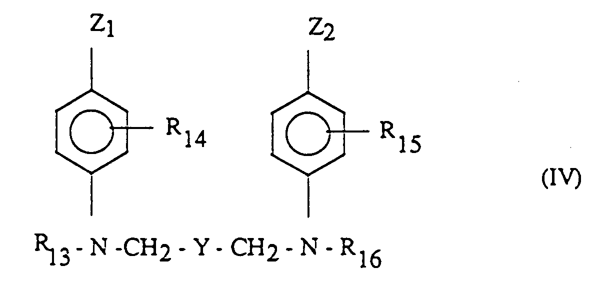 dans laquelle :
Z₁ et Z₂, identiques ou différents, représentent des groupements hydroxyle ou NHR₁₇, où R₁₇ désigne un atome d'hydrogène ou un radical alkyle inférieur ;
R₁₄ et R₁₅, identiques ou différents, représentent soit des atomes d'hydrogène, soit des atomes d'halogène, soit encore des radicaux alkyle ;
R₁₃ et R₁₆, identiques ou différents, représentent un atome d'hydrogène, un radical alkyle, hydroxyalkyle ou aminoalkyle, dont le reste amino peut être substitué; Y représente un radical pris dans le groupe constitué par les radicaux suivants :
   -(CH₂)ₙ-, -(CH₂)ₘ-O-(CH₂)ₘ-,
   -(CH₂)_{q}-CHOH-(CH₂)_{q}-, 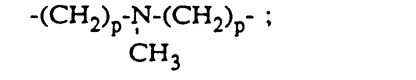 dans lesquels
      n est un nombre entier compris entre 0 et 8 et m, q et p sont des nombres entiers compris entre 0 et 4, cette base pouvant se présenter également sous forme de ses sels d'addition avec des acides.

Les radicaux alkyle ou alcoxy ci-dessus indiqués désignent de préférence un groupement ayant 1 à 4 atomes de carbone et notamment méthyle, éthyle, propyle, méthoxy et éthoxy.

Parmi les composés de formule (IV) on peut citer le N,N'-bis-(β-hydroéthyl)N,N'-bis-(4'-aminophényl) 1,3-diamino 2-propanol, la N,N'-bis-(β-hydroxyéthyl)N,N'-bis-(4'-aminophényl)éthylènediamine, la N,N'-bis-(4-aminophényl)tétraméthylènediamine, la N,N'-bis-(β-hydroxyéthyl) N,N'-bis-(4-aminophényl)tétraméthylènediamine, la N,N'-bis-(4-méthylaminophényl)tétraméthylènediamine, la N,N'-bis-(éthyl) N,N'-bis-(4'-amino 3'-méthylphényl)éthylènediamine.

Parmi les orthoaminophénols, on peut citer plus particulièrement le 1-amino-2-hydroxybenzène, le 6-méthyl 1-hydroxy 2-aminobenzène, le 4-méthyl 1-amino 2-hydroxybenzène, le 4-acétylamino 1-amino 2-hydroxybenzène.

Les composés de formule (I) sont appliqués sur les fibres kératininiques et en particulier les cheveux humains au moyen de compositions tinctoriales qui constituent un autre objet de l'invention.

Les compositions conformes à l'invention contiennent dans un milieu approprié pour la teinture au moins une métaphénylènediamine soufrée définie ci-dessus. Les compositions préférées contiennent au moins une métaphénylènediamine soufrée définie ci-dessus, en association avec au moins un présurseur de colorant d'oxydation tel que défini ci-dessus.

Les compositions tinctoriales conformes à l'invention peuvent également contenir en plus du coupleur répondant à la formule (I) définie ci-dessus, d'autres coupleurs connus en eux-mêmes, tels que les métadiphénols, les métaaminophénols, les métaphénylènediamines différentes de celles de formule (I) ci-dessus, les métaacylaminophénols, les métauréidophénols, les métacarbalcoxyaminophénols, l'α-naphtol, les dérivés indoliques, les coupleurs possédant un groupement méthylène actif, tel que les composés β-cétoniques, les pyrazolones.

Parmi ces coupleurs on peut plus particulièrement citer, le 2, 4-dihydroxyphénoxyéthanol, le 2,4-dihydroxyanisole, le métaaminophénol, le monométhyléther de résorcine, la résorcine, la 2-méthyl-résorcine, le 2-méthyl 5-aminophénol, le 2-méthyl 5-N-(β-hydroxyéthyl) aminophénol, le 2-méthyl 5-N-(β-mésylaminoéthyl)aminophénol, le 2,6-diméthyl 3-aminophénol, la 6-hydroxybenzomorpholine, le 2,4-diaminoanisole, le 2,4-diaminophénoxyéthanol, la 6-aminobenzomorpholine, le [2-N-(β-hydroxyéthyl) amino 4-amino]-phénoxyéthanol, le 2-amino 4-N-(β-hydroxyéthyl) amino anisole, le (2,4-diamino)phényl-β,γ-dihydroxypropyléther, la 2,4-diaminophénoxyéthylamine, le 1,3-diméthoxy 2,4-diaminobenzène, le 1,3,5-triméthoxy 2,4-diaminobenzène, le 1-amino 3,4-méthylènedioxybenzène, le 1-hydroxy 3,4-méthylènedioxybenzène, le 2-chloro 6-méthyl 3-aminophénol, le 2-méthyl 3-aminophénol, le 2-chloro résorcinol, la 6-méthoxy 3-hydroxyéthylaminoaniline, le 1-éthoxy 2-bis(β-hydroxyéthyl)amino 4-aminobenzène, le 3-diéthylaminophénol, le 1,3-dihydroxy 2-méthylbenzène, le 1-hydroxy 2,4-dichloro 3-aminobenzène, le 4,6-di(hydroxyéthoxy) 1,3-diaminobenzène, le 4-méthyl 6-éthoxy 1,3-diaminobenzène, le 4-chloro 6-méthyl 3-aminophénol, le 6-chloro 3-trifluoroéthylaminophénol, et leurs sels.

On peut rajouter à ces compositions, comme cela est bien connu dans l'état de la technique, notamment en vue de nuancer ou d'enrichir en reflet les colorations apportées par les précurseurs de colorants d'oxydation, des colorants directs tels que des colorants azoïques, anthraquinoniques ou les dérivés nitrés de la série benzénique.

L'ensemble des précurseurs de colorants par oxydation de type para et/ou ortho, ainsi que les coupleurs utilisés dans les compositions tinctoriales conformes à l'invention, représente de préférence de 0,3 à 7 % en poids par rapport au poids de la dite composition. La concentration en composés métaphénylènediamines soufrées de formule (I) peut varier entre 0,05 et 3,5 % en poids du poids total de la composition.

Les compositions tinctoriales conformes à l'invention contiennent également dans leur forme de réalisation préférée, des agents tensio-actifs anioniques, cationiques, non-ioniques, amphotères ou leurs mélanges, bien connus dans l'état de la technique.

Ces agents tensio-actifs sont présents dans les compositions conformes à l'invention dans des proportions comprises entre 0,5 et 55 % en poids, et de préférence entre 2 et 50 % en poids par rapport au poids total de la composition.

Ces compositions peuvent également contenir des solvants organiques pour solubiliser les composants qui ne seraient pas suffisamment solubles dans l'eau. Parmi ces solvants, on peut citer à titre d'exemple, les alcanols inférieurs en C₁-C₄, tels que l'éthanol et l'isopropanol; le glycérol; les glycols ou éthers de glycols comme le 2-butoxyéthanol, l'éthylèneglycol, le propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants sont présents de préférence dans des proportions comprises entre 1 et 40 % en poids, et en particulier entre 5 et 30 % en poids par rapport au poids total de la composition.

Les agents épaississants que l'on peut ajouter dans les compositions conformes à l'invention peuvent être choisis parmi l'alginate de sodium, la gomme arabique, les polymères d'acide acrylique éventuellement réticulés, les dérivés de cellulose, les hétérobiopolysaccharides tels que la gomme de xanthane, on peut également utiliser des agents épaississants minéraux tels que la bentonite.

Ces agents épaississants sont présents de préférence dans des proportions comprises entre 0,1 et 5 %, et en particulier entre 0,2 et 3 % en poids par rapport au poids total de la composition.

Les agents antioxydants qui peuvent être présents dans les compositions sont choisis en particulier parmi le sulfite de sodium, l'acide thioglycolique, le bisulfite de sodium, l'acide déhydroascorbique, l'hydroquinone et l'acide homogentisique. Ces agents antioxydants sont présents dans la composition dans des proportions comprises entre 0,05 et 1,5 % en poids par rapport au poids total de la composition.

Le pH de ces compositions est compris entre 3 et 10,5. Il est ajusté à la valeur désirée à l'aide d'agents alcalinisants bien connus de l'état de la technique, tels que l'ammoniaque, les carbonates alcalins, les alcanolamines tels que les mono-, di- et triéthanolamines ainsi que leurs dérivés ou les hydroxydes de sodium et de potassium, ou d'agents acidifiants classiques, tels que les acides minéraux ou organiques, tels que les acides chlorhydrique, tartrique, citrique, et phosphorique.

Ces compositions peuvent également contenir d'autres adjuvants cosmétiquement acceptables, tels que par exemple des agents de pénétration, des agents séquestrants, des parfums, des tampons, etc.

Les compositions conformes à l'invention peuvent se présenter sous des formes diverses, telles que sous forme de liquide, de crème, de gel ou sous toute autre forme appropriée pour réaliser une teinture des fibres kératiniques et notarnment des cheveux humains. Ces compositions peuvent être conditionnées en flacons aérosols en présence d'un agent propulseur et former des mousses.

Les composés de formule (I) sont utilisés conformément à l'invention selon un procédé comprenant l'application sur les fibres kératiniques du composé de formule (I) et de précurseurs de colorants d'oxydation ortho et/ou para en présence d'un agent oxydant.

Les compositions tinctoriales conformes à l'invention contenant un précurseur de colorant par oxydation du type para et/ou ortho et un coupleur de formule (I), sont utilisées suivant un procédé mettant en oeuvre la révélation par un agent oxydant.

Conformément à ce procédé on mélange, au moment de l'emploi, la composition tinctoriale décrite ci-dessus avec une solution oxydante en une quantité suffisante pour pouvoir développer une coloration, puis on applique le mélange obtenu sur les fibres kératiniques et en particulier les cheveux humains.

Le pH de la composition appliquée sur les cheveux varie de préférence entre 2 et 13. Il est ajusté à la valeur désirée à l'aide d'agents alcalinisants bien connus de l'état de la technique, tels que l'ammoniaque, les carbonates alcalins, les alcanolamines comme la mono-, la di- et la triéthanolamine, ainsi que leurs dérivés ou les hydroxydes de sodium ou de potassium ou d'agents acidifiants classiques, tels que les acides minéraux ou organiques, tels que les acides chlorhydrique, tartrique, citrique, phosphorique et sulfonique. La solution oxydante contient à titre d'agent oxydant, l'eau oxygénée, le peroxyde d'urée, des persels, tels que le persulfate d'ammonium des peracides organiques et leurs sels ou des bromates de métaux alcalins. On utilise de préférence une solution d'eau oxygénée à 20 volumes.

Le mélange obtenu est appliqué sur les cheveux et on laisse poser pendant 10 à 40 minutes, de préférence 15 à 30 minutes, après quoi on les rince, les lave au shampooing, on les rince à nouveau et on les sèche.

Le coupleur de formule (I) définie ci-dessus, peut également être mis en oeuvre dans un procédé à plusieurs étapes, consistant dans l'une des étapes, à appliquer le précurseur de colorant d'oxydation du type ortho et/ou para ou leur mélange et, dans une autre étape, à appliquer une composition tinctoriale contenant le coupleur de formule (I).

L'agent oxydant peut être introduit, juste avant l'application, dans la composition appliquée dans le deuxième temps ou bien être appliqué sur les fibres kératiniques elles-mêmes, dans un troisième temps, les conditions de pose, de pH, de lavage et de séchage étant identiques à celles indiquées ci-dessus.

Un autre objet de l'invention est constitué par de nouvelles métaphénylènediamines soufrées qui répondent à la formule : 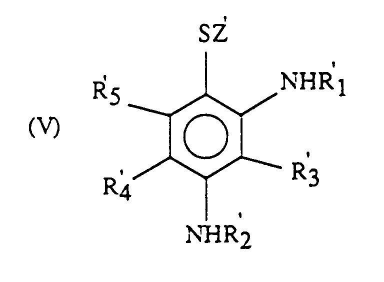 dans laquelle :
Z' représente un radical alkyle en C₁-C₁₈, un radical aralkyle dans lequel le radical alkyle est en C₁-C₆, un radical monohydroxyalkyle en C₁-C₆ ou polyhydroxyalkyle C₂-C₆, un radical aryle, un radical fluoroalkyle en C₁-C₄,un radical aminoalkyle de formule : 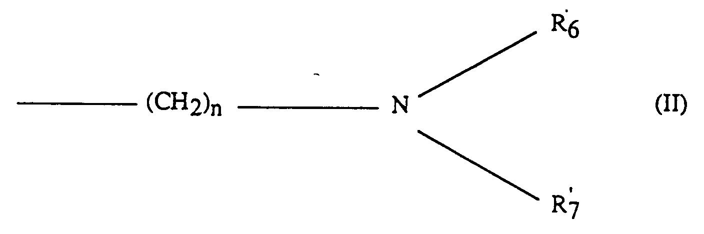 dans laquelle
   n est un nombre entier compris entre 1 et 6 inclus; R'₆ et R'₇, identiques aux différents, représentent un atome d'hydrogène ou un radical alkyle en C₁-C₄, hydroxyalkyle en C₁-C₄, acyle en C₁-C₆;
R'₁ et R'₂, identiques ou différents, représentent un atome d'hydrogène, un radical alkyle en C₁-C₆, un radical monohydroxyalkyle en C₁-C₆, polyhydroxyalkyle en C₂-C₆, monocarbamylalkyle en C₁-C₆, dialkyl-carbamyle en C₁-C₆, aminoalkyle en C₁-C₆, acylaminoalkyle (C₁-C₄), carbalcoxy (C₂-C₆) alkyle (C₁-C₄), carbamyle ou monoalkyl en C₁-C₆ carbamyle, fluoroalkyle en C₁-C₄ ;
R'₃ représente un atome d'hydrogène, un radical alkyle en C₁-C₄;
R'₄ et R'₅, identiques ou différents, représentant un atome d'hydrogène, un halogène, un radical alcoxy en C₁-C₄, hydroxyalkyle en C₁-C₄, un radical -SR avec R représentant un radical hydroxyalkyle en C₁-C₄, polyhydroxyalkyle en C₂-C₆, aralkyle dans lequel le radical alkyle est en C₁-C₆, un radical aryle, un radical aminoalkyle de formule : 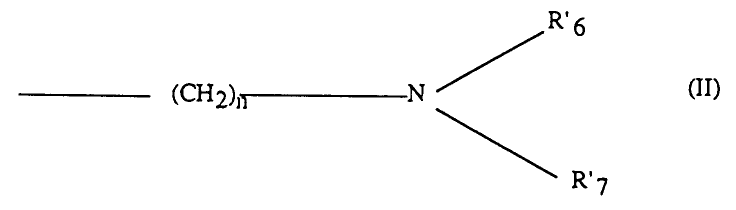 dans laquelle
   n est un nombre entier compris entre 1 et 6 inclus; R'₆ et R'₇ identiques ou différents, représentent un atome d'hydrogène ou un radical alkyle en C₁-C₄, hydroxyalkyle en C₁-C₄ ou acyle en C₁-C₆, sous réserve que R'₄ et R'₅ ne désignent pas simultanément un atome d'hydrogène;
et lorsque un des R'₄ ou R'₅ désigne un atome de chlore, R'₃ représente un radical alkyle.
Parmi les composés répondant à la formule (V) définie ci-dessus on peut citer de préférence :
le 4-méthoxy-6-β-acétylaminoéthylthio -1,3 diaminobenzène dénommé selon la nomenclature IUPAC N-[2-(2,4-diamino-5-méthoxy-phénylsulfanyl-éthyl]-acétamide
le 4-méthoxy-6-méthylthio-1,3-diaminobenzène dénommé selon la nomenclature IUPAC 4-méthoxy-6-méthylsulfanyl-benzène-1,3-diamine
le 5-chloro-2-méthyl-4-β-acétylamonoéthylthio-1,3-diaminobenzène dénommé selon la nomenclature IUPAC N[2-[2,4-diamino 6-chloro 3-méthylphénylsulfanyl)éthyl] acétamide.

Le 4,6-bis-hydroxyéthyl thio-1,3-diaminobenzène dénommé selon la nomenclature IUPAC 2-[2,4-diamino-5-(2-hydroxy-éthylsulfanyl)-phénylsulfanyl]-éthanol

Les métaphénylènediamines soufrées de formule (V) ou leurs sels peuvent être préparées selon un procédé en plusieurs étapes.

Selon un premier procédé et dans une première étape, on fait réagir en présence d'une base telle que la potasse ou le carbonate de potassium, le 1,3-dichloro 4,6-dinitrobenzène sur un thiol de formule (VI) :

Z₁-SH (VI)

dans laquelle
Z₁ représente un radical alkyle en C₁-C₁₈, aralkyle dans lequel le radical alkyle est en C₁-C₆, monohydroxyalkyle en C₁-C₆, polyhydroxyalkyle en C₂-C₆, un radical aryle ou un groupement de formule (VII) 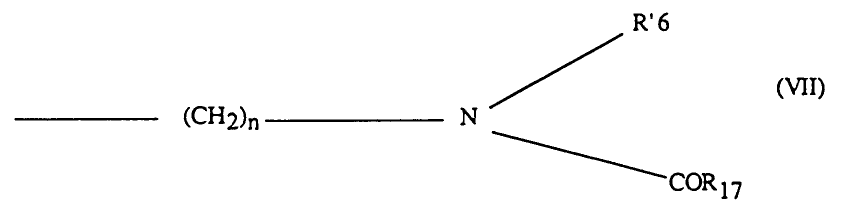 dans laquelle
   R'₆ et n ont les significations indiquées précédemment dans la formule (V); et R₁₇ représente un atome d'hydrogène ou un radical alkyle en C₁-C₃ ;
dans une deuxième étape, on réduit les substituants nitro du composé de formule (VIII) : 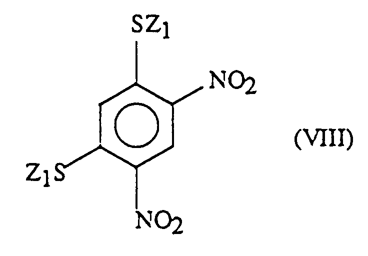 obtenu précédemment pour préparer un composé répondant à la formule (IX) : 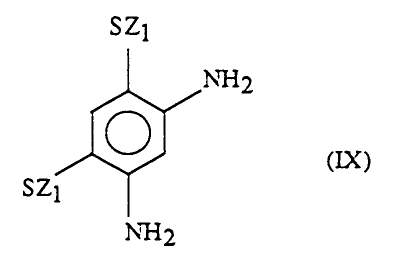 dans laquelle
Z₁ a la signification indiquée ci-dessus ;
éventuellement, dans une troisième étape, et suivant le composé métaphénylènediamine soufrée de formule (I) que l'on souhaite obtenir, on effectue
a) soit une mono-substitution des amines aromatiques pour obtenir un composé de formule (V) dans laquelle R'₁ et/ou R'₂ sont différents de H;
b) soit une hydrolyse acide du composé (IX) dans lequel Z' représente un groupement de formule (VII) pour obtenir le composé de formule (X). 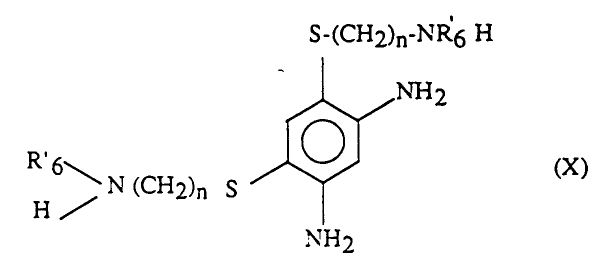 dans laquelle
   R'₆ et n ont les significations indiquées ci-dessus, R'₆ ne désignant toutefois pas de radical acyle en C₁-C₆,
   les amines nucléaires pouvant être ensuite monosubstituées.
c) soit on effectue au préalable une substitution de l'amine extranucléaire du composé de formule (IX) pour obtenir le composé de formule (XI) 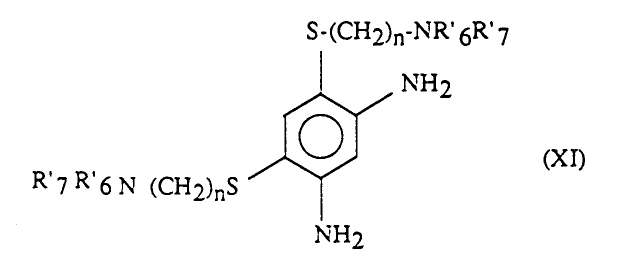 dans laquelle
   R'₆ et R'₇ et n ont les significations indiquées ci-dessus,
   les amines nucléaires pouvant être ensuite monosubstituées.
Selon un second procédé, et dans une première étape on fait réagir un composé substitué de formule (XII) 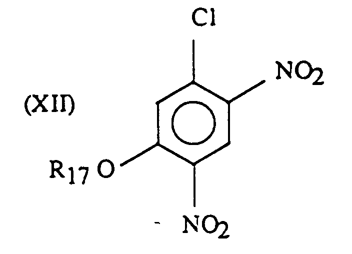 dans laquelle
R₁₇ représente un groupement alkyle en C₁-C₄ sur un thiol de formule :

   Z₁ -SM (XIII)

   dans laquelle,
   M est un métal alcalin et Z₁ a les significations indiquées ci-dessus.

Dans une deuxième étape, on réduit le substituant nitro du composé de formule (XIV) 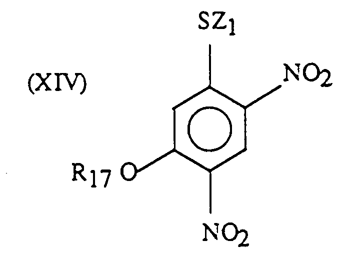 obtenu précédemment pour préparer le composé de formule (XV) 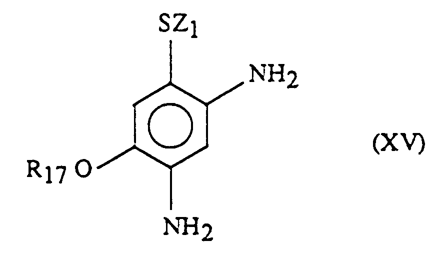 dans laquelle
R₁₇ et Z₁ ont les significations indiquées ci-dessus ;
éventuellement, dans une troisième étape, et suivant la métaphénylènediamine soufrée de formule (V) que l'on souhaite obtenir, on effectue une monosubstitution de l'amine aromatique pour obtenir un composé de formule (V) dans laquelle R'₁ ou R'₂ est différent de H.

Selon un troisième procédé et dans une première étape on fait réagir un 2,4-dinitrobenzène polysubstitué de formule (XVI) 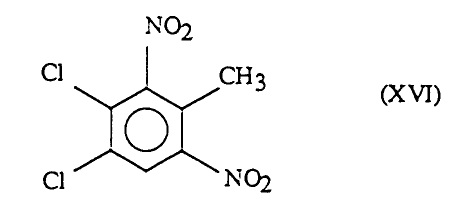 sur un thiol de formule :

Z₁-SM (XIII)

dans laquelle,
M est un métal alcalin et Z₁ a les significations indiquées ci-dessus.

Dans une deuxième étape, on réduit les substituants nitro du composé de formule (XVII) : 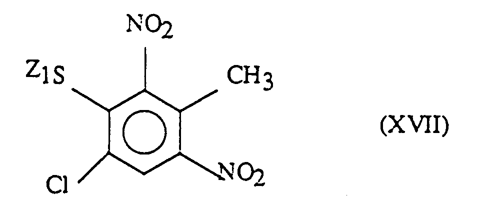 obtenu précédemment pour préparer un composé répondant à la formule (XVIII). 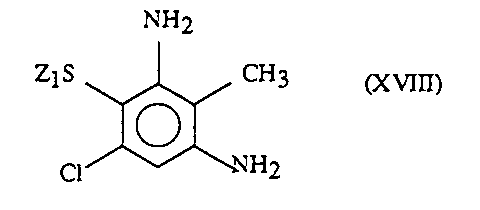 dans laquelle
Z₁ a les significations indiquées ci-dessus.

La réduction des groupes nitro s'effectue de préférence en utilisant du fer en milieu acétique ou alors par le cyclohexène en présence d'un catalyseur palladium-charbon ou encore par le zinc en poudre en présence d'éthanol et de chlorure d'ammonium ou par tout autre procédé de réduction classique.

La substitution des amines aromatiques ou de l'amine extra-nucléaire peut être effectuée en faisant réagir, par exemple, le bromure d'éthyle, la bromhydrine du glycol, l'éthyl-chloroformiate, la β-chloracétamide, ou l'anhydride acétique.

Les exemples qui suivent sont destinés à illustrer l'invention sans pour autant présenter un caractère limitatif.

### Exemple de préparation 1 :

### Préparation du dichlorhydrate, monohydrate de 4-méthoxy 6-methylthio 1,3- diaminobenzène dénommé selon la nomenclature IUPAC dichlorhydrate de 4-méthoxy 6-méthylsulfanyl-benzène 1,3-diamine, monohydrate.

### 1ère étape : Synthèse du (5-méthoxy 2,4-dinitrophényl)-méthyl-sulfane.

A une suspension de thiométhylate de sodium (0,15 mole) dans 120 ml de diméthoxyéthane à température ambiante, on ajoute goutte à goutte en une heure une solution de 23,2 g (0,1 mole) de 1-chloro 5-méthoxy-2,4-dinitro-benzène dans 60 ml de diméthoxyéthane.

La réaction est exothermique : on maintient la température entre 23 et 25°.

A la fin de la coulée, on agite la suspension pendant une demie heure et puis on verse dans 60 ml d'eau glacée. Le précipité cristallisé est essoré, réempaté dans l'eau, lavé à l'isopropanol et recristallisé du diméthoxyéthane bouillant.

On obtient des cristaux jaunes (16,9 g) fondant à 161°C et dont l'analyse élémentaire calculée pour C₈H₈N₂O₅ S est :

| | C % | H % | N % | O % | S % |
|---|---|---|---|---|---|
| Calculé | 39,34 | 3,30 | 11,47 | 32,76 | 13,13 |
| Trouvé | 39,54 | 3,28 | 11,44 | 32,71 | 13,20 |

### 2ème étape : réduction.

On chauffe au reflux de l'alcool un mélange de 2,2 g de chlorure d'ammonium, 16,5 ml d'eau, 140 ml d'alcool à 96° et 41 g de zinc en poudre fine. On ajoute par portions le (5-méthoxy 2,4-dinitrophényl)méthyl-sulfane obtenu à l'étape 1 (11,0 g-0,045 mole) de façon à maintenir le reflux sans chauffage. La réaction est exothermique.

A la fin de l'addition le chauffage au reflux est prolongé pendant une demi-heure. Le milieu réactionnel est filtré bouillant sur 17 ml d'alcool absolu chlorhydrique environ 6 N.

Par refroidissement du filtrat, le dichlorhydrate de 4-méthoxy 6-méthylsulfanyl-benzène-1,3-diamine, monohydrate cristallise.

Après essorage et séchage sous vide sur potasse on obtient 7,0 g de cristaux blancs fondant avec décomposition à 198-203°C et dont l'analyse élémentaire calculée pour C₈H₁₆Cl₂ N₂O₂S est :

| | C % | H % | N % | O % | S % | cl % |
|---|---|---|---|---|---|---|
| Calculé | 34,92 | 5,86 | 10,18 | 11,63 | 11,65 | 25,77 |
| Trouvé | 35,23 | 5,87 | 10,23 | 11,30 | 11,81 | 25,63 |

### Exemple de préparation 2 :

### Préparation du dichlorhydrate, monohydrate de 4-méthoxy 6-β-acétylaminoéthylthio 1,3-diaminobenzène dénommé selon la nomenclature IUPAC dichlorhydrate de N-[2-(2,4-diamino-5-méthoxy-phénylsulfanyl)-éthyl]-acétamide monohydrate.

### 1ère étape : Synthèse du N-[2-(5-méthoxy 2,4-dinitro-phénylsulfanyl)-éthyl]-acétamide.

On dissout 10 g de potasse en poudre dans une solution de 19,0 g (0,15 mole) de N-(2-mercapto-éthyl)-acétamide dans 100 ml de diméthoxyéthane chauffée à 40°C.

Après refroidissement à 15°C on coule goutte-à-goutte, en trente minutes, une solution de 23,2 g (0,1 mole) de 1-chloro-5-méthoxy-2,4-dinitro-benzène dans 60 ml de diméthoxyéthane, en maintenant la température entre 15 et 20°C.

La suspension est agitée pendant 1 heure puis est versée dans 500 ml d'eau glacée.

Le précipité cristallisé est essoré, réempaté dans l'eau puis dans l'alcool isopropylique et recristallisé de l'alcool à 96° bouillant.

On obtient 23,2 g de cristaux jaunes fondant à 185°C et dont l'analyse élémentaire calculée pour C₁₁H₁₃N₃O₆ S est :

| | C % | H % | N % | O % | S % |
|---|---|---|---|---|---|
| Calculé | 41,90 | 4,16 | 13,33 | 30,45 | 10,17 |
| Trouvé | 41,94 | 4,15 | 13,38 | 30,54 | 10,09 |

### 2ème étape : Réduction

La réduction est effectuée selon le mode opératoire décrit pour l'exemple 1 étape 2.

On obtient des cristaux blancs de dichlorhydrate de N-[2-(2,4-diamino 5-méthoxy-phénylsulfanyl)-éthyl]-acétamide monohydrate fondant avec décomposition à 183-187°C et dont l'analyse élémentaire calculée pour C₁₁H₂1N₃O₃S cl est :

| | C % | H % | N % | O % | S % | cl % |
|---|---|---|---|---|---|---|
| Calculé | 38,16 | 6,11 | 12,13 | 13,86 | 9,26 | 20,48 |
| Trouvé | 37,83 | 6,48 | 11,93 | 14,12 | 9,25 | 20,37 |

### Exemple de préparation 3 :

### Préparation du 4,6-bis-hydroxyéthylthio 1,3-diaminobenzène dénommé selon la nomenclature IUPAC 2-[2,4-diamino 5(2-hydroxyéthylsulfanyl)-phénylsulfanyl]-éthanol.

### 1ère étape : Synthèse du 2-[5-(2-hydroxy-éthylsulfanyl)2,4-dinitro-phénylsulfanyl]-éthanol.

On chauffe à 60°C un mélange de 34,5 g de carbonate de potassium, de 8 g de 2-mercaptoéthanol et de 100 ml de dioxane.

On ajoute 11,9 g (0,05) de 1,5 dichloro-2,4-dinitro-benzène et prolonge le chauffage 1 h à 60° puis 1 h à 100°C.

Le milieu réactionnel est versé dans 500 ml d'eau glacée. Le précipité cristallisé est essoré, réempaté dans l'eau et séché sous vide sur anhydride phosphorique.

Après recristallisation, on obtient 9,0 g de cristaux jaune orangé fondant à 149° C.

### 2ème étage : Réduction

La réduction est effectuée selon le mode opératoire décrit pour l'exemple 1, étape 2.

On obtient des cristaux blancs (81 %) de 2-[2,4-diamino-5-(2-hydroxy-éthylsulfanyl)-phénylsulfanyl]-éthanol fondant à 144°C et dont l'analyse élémentaire calculée pour C₁₀H₁₆N₂O₂S₂ est :

| | C % | H % | N % | O % | S % |
|---|---|---|---|---|---|
| Calculé | 46,13 | 6,19 | 10,76 | 12,29 | 24,63 |
| Trouvé | 46,21 | 6,07 | 10,67 | 12,42 | 24,57 |

### Exemple de préparation 4 :

### Préparation du 5-chloro 2-méthyl 4-β-acétaminoéthylthio 1,3-diaminobenzène dénommé selon la nomenclature IUPAC N-[2-(2,4-diamino 6-chloro 3-méthyl-phénylsulfanyl)-éthyl]-acétamide.

### 1ère étape: Synthèse du N-[2-(6-chloro 3-méthyl 2,4-dinitrophénylsulfanyl)-éthyl]-acétamide.

Cette synthèse est effectuée selon le mode opératoire décrit pour l'exemple 2, étape 1. En partant de 30,1 g (0,12 mole) de 1,2-dichloro-4-méthyl 3,5-dinitro-benzène, on obtient 15,5 g de cristaux jaune pâle fondant à 152°C (recristallisés de l'acétate d'éthyle) et dont l'analyse élémentaire calculée pour C₁₁H₁₂Cl N₃OS est :

| | C % | H % | N % | O % | S % | Cl % |
|---|---|---|---|---|---|---|
| Calculé | 39,59 | 3,62 | 12,59 | 23,97 | 9,61 | 10,62 |
| Trouvé | 39,67 | 3,62 | 12,63 | 23,84 | 9,69 | 10,56 |

### 2ème étape : Réduction

La réduction est effectuée selon le mode opératoire décrit pour l'exemple 1, étape 2.

On obtient des cristaux blancs (recristallisés de l'éthanol 96°) de N-[2-(2,4-diamino 6-chloro 3-méthyl-phénylsulfanyl)-éthyl]acétamide fondant à 111°C et dont l'analyse élémentaire calculée pour C₁₁H₁₆ Cl N₃ OS est :

| | C % | H % | N % | O % | S % | Cl % |
|---|---|---|---|---|---|---|
| Calculé | 48,26 | 5,89 | 15,35 | 5,84 | 11,71 | 12,95 |
| Trouvé | 48,31 | 5,92 | 15,27 | 5,92 | 11,69 | 12,87 |

### Exemples de compositions

### EXEMPLE 1

| | |
|---|---|
| -Dichlorhydrate, monohydrate de 4-méthoxy 6-β-acétylaminoéthylthio 1,3-diaminobenzène | 1,038 g |
| -Dichlorhydrate de 2,6-diméthyl paraphénylènediamine | 0,627 g |
| -Alcool oléique polyglycérolé à 2 moles de glycérol | 4 g |
| -Alcool oléique polyglycérolé à 4 moles de glycérol | 5,7 g |
| -Acide oléique | 3 g |
| -Amine oléique oxyéthylénée à 2 moles d'oxyde d'éthylène commercialisé sous la dénomination ETHOMEEN O12 par la Société AKZO | 7 g |
| -Sel de sodium du laurylamino succinamate de diéthylaminopropyle | 3 g MA |
| -Alcool oléique | 5 g |
| -Diéthanolamide d'acide oléique | 12 g |
| -Propylène glycol | 3,5 g |
| -Alcool éthylique | 7 g |
| -Dipropylène glycol | 0,5 g |
| -Monométhyléther de propylène glycol | 9 g |
| -Métabisulfite de sodium en solution aqueuse à 35 % | 0,45 g MA |
| -Acétate d'ammonium | 0,8 g |
| -Antioxydant, séquestrant | q.s |
| -Parfum, conservateur | q.s |
| -Monoéthanolamine q.s. pH : 9,8 | |
| -Eau déminéralisée q.s.p. | 100 g |

Au moment de l'emploi, on mélange la composition ci-dessus, poids pour poids, avec de l'eau oxygénée à 20 volumes dont le pH est ajusté entre 1 et 1,5 par addition d'acide orthophosphorique. Le pH du mélange est égal à 6,5. Celui-ci est appliqué sur des cheveux gris à 90 % de blancs pendant 30 minutes à température ambiante. Les cheveux sont ensuite rincés, lavés au shampooing, rincés une nouvelle fois puis séchés. **Ils sont colorés en bleu.**

### EXEMPLE 2

Il est similaire à l'exemple 1 à la seule différence que l'on remplace les 1,038 g de dichlorhydrate, monohydrate de 4-méthoxy-6-β-acétylaminoéthylthio 1,3-diaminobenzène par 0,821 g de 5-chloro 2-méthyl 4-β-acétylaminoéthylthio 1,3-diaminobenzène.

Les conditions de teinture sont identiques à celles décrites à l'exemple 1. Les cheveux gris à 90 % de blancs sont colorés en **doré mât**.

### EXEMPLE 3

On prépare la composition tintoriale suivante :

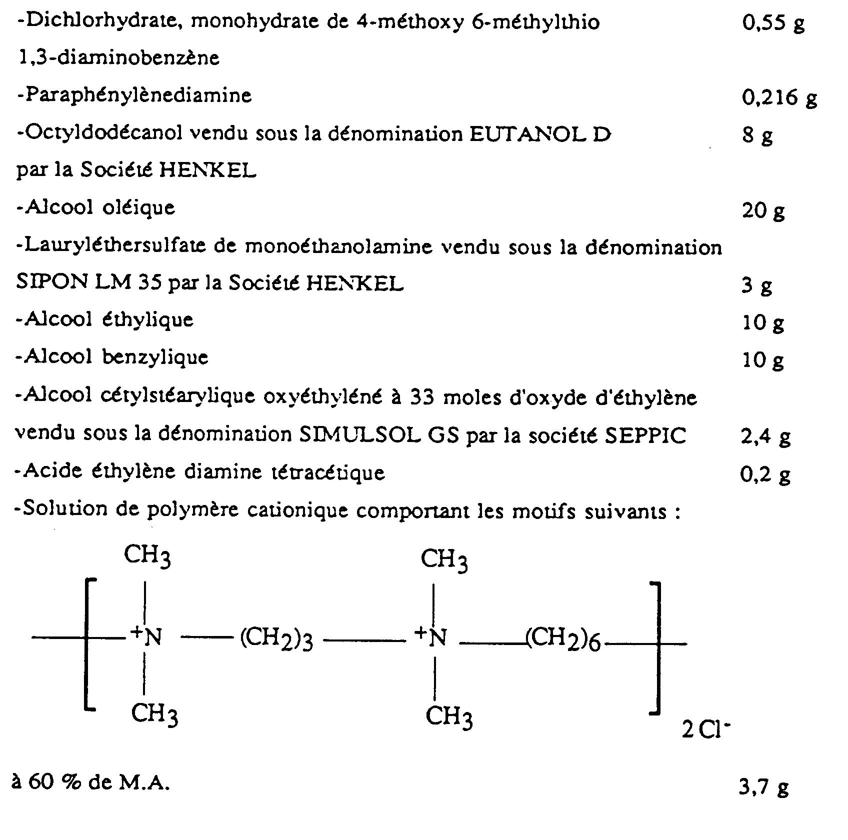

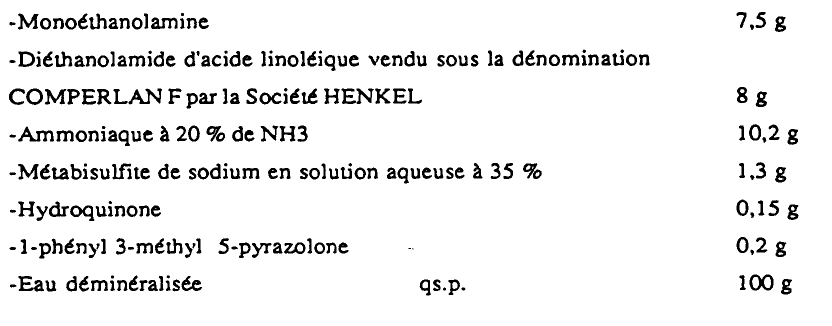

Cette composition est mélangée au moment de l'emploi, poids pour poids, avec de l'eau oxygénée à 20 volumes dont le pH est égal à 3. Le pH du mélange est égal à 9,5. Celui-ci est appliqué sur des cheveux gris permanentés pendant 30 minutes à température ambiante. Les cheveux sont ensuite rincés, lavés au shampooing et séchés. **Ils sont colorés en gris bleuté mât**.

## Revendications

1. Utilisation pour la teinture des fibres kératiniques, et en particulier les cheveux humains, d'au moins une métaphénylènediamine soufrée de formule générale : 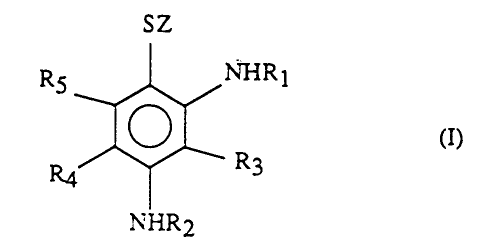 dans laquelle :
Z représente un radical alkyle en C₁-C₁₈, un radical aralkyle dans lequel le radical alkyle est en C₁-C₆, un radical monohydroxyalkyle en C₁-C₆ ou polyhydroxyalkyle en C₂-C₆, un radical aryle, un radical fluoroalkyle en C₁-C₄, un radical aminoalkyle de formule : 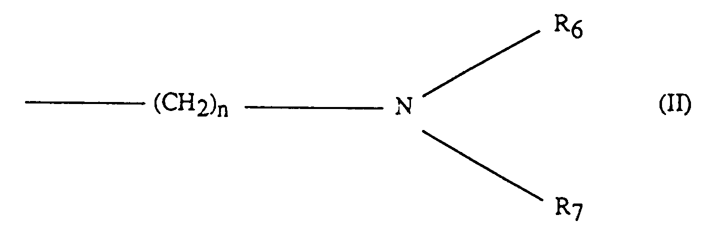 dans laquelle
n est un nombre entier compris entre 1 et 6 inclus, R₆ et R₇, identiques ou différents, représentent un atome d'hydrogène ou un radical alkyle en C₁-C₄, hydroxyalkyle en C₁-C₄, acyle en C₁-C₆;
R₁ et R₂, identiques ou différents, représentent un atome d'hydrogène, un radical alkyle en C₁-C₆, un radical monohydroxyalkyle en C₁-C₆, polyhydroxyalkyle en C₂-C₆, monocarbamylalkyle en C₁-C₆, dialkylcarbamyle (C₁-C₆), aminoalkyle en C₁-C₆, acylaminoalkyle en C₁-C₄, carbalcoxy en C₂-C₆ alkyle (C₁-C₄), carbamyle ou monoalkyl en C₁-C₆ carbamyle, fluoroalkyle en C₁-C₄
R₃ représente un atome d'hydrogène, un radical alkyle en C₁-C₄;
R₄ et R₅, identiques ou différents, représentent un atome d'hydrogène, un radical alkyle en C₁-C₄, un radical alcoxy en C₁-C₄, hydroxyalkyle en C₁-C₄, un halogène, un radical -SZ où Z a les significations indiquées ci-dessus, au moins un des radicaux R₃, R₄ ou R₅ est différent d'un atome d'hydrogène et leurs sels d'acides correspondants.

2. Utilisation selon la revendication 1, caractérisée par le fait que la métaphénylènediamine soufrée de formule (I) est mise en oeuvre en présence de précurseurs de colorants d'oxydation de type ortho et/ou para.

3. Utilisation selon la revendication 1 ou 2, caractérisée par le fait que dans le composé de formule (I), Z désigne un radical méthyle, éthyle, propyle, butyle, dodécyle, hexadécyle, le radical benzyle, le radical phenyle, un radical mono ou polyhydroxyalkyle choisi parmi:
-CH₂-CH₂-OH, -CH₂-CHOH-CH₂-OH, -CH₂-CHOH-CH₃, un radical aminoalkyle choisi parmi :
-CH₂-CH₂NH₂, -CH₂-CH₂-NHCH₃,
un radical acylaminoalkyle choisi parmi :
-CH₂-CH₂-NH-COCH₃ ; ou 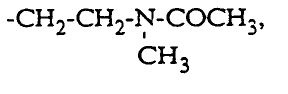 et lorsque R₆ ou R₇ désignent un radical acyle, celui-ci représente un groupement formyle, acétyle ou propionyle.

4. Utilisation selon l'une quelconque des revendications 1 à 3 caractérisée en ce que les composés de formule (I) sont choisis parmi les composés suivants :
le 2-méthyl-4-méthylthio-1,3-diaminobenzène
le 2-éthyl-4-méthylthio-1,3-diaminobenzène
le 4-chloro-6-propylthio-1,3 diamino benzène
le 4,6-bis-méthylthio-1,3-diaminobenzène
le 4,6-bis-éthylthio-1,3-diaminobenzène
le 4,6-bis-trifluorométhylthio-1,3-[N,N'-bis-(2',2',2'-trifluoroéthyl)] diamino benzène
le 4-méthyl-6-méthylthio -1,3-diaminobenzène
le 4-éthylthio -6-méthyl-1,3-diamino benzène
le 4-carboxyéthylthio 6-carboxyméthylthio-1,3-diaminobenzène
le 4,6-bis-carboxyéthylthio 1,3-diaminobenzène
le 2-méthyl 4,6-bis-méthylthio 1,3-diaminobenzène
le 4,6-bis-éthylthio-2-méthyl 1,3-diaminobenzène
le 4,6-bis-propylthio-2-méthyl 1,3-diaminobenzène
le 4-méthoxy 6-β-acétylaminoéthylthio-1,3-diaminobenzène
le 4-méthoxy 6-méthylthio 1,3-diaminobenzène
le 5-chloro 2-méthyl 4-β-acétylaminoéthylthio 1,3-diaminobenzène
le 4,6-bis-hydroxyéthyl thio 1,3-diaminobenzène
et leurs sels d'acides.

5. Utilisation selon l'une quelconque des revendications 1 à 4, caractérisé par le fait que les sels d'acides correspondants sont choisis parmi les chlorhydrates, les sulfates ou les bromhydrates.

6. Utilisation selon l'une quelconque des revendications 2 à 5, caractérisée par le fait que les précurseurs de colorants d'oxydation sont choisis parmi les paraphénylènediamines, les paraaminophénols, les précurseurs hétérocycliques para dérivés de la pyridine, de la pyrimidine, ou du pyrazole, les orthoaminophénols et les bis-phénylalkylènediamines.

7. Composition tinctoriale pour fibres kératiniques, et en particulier les cheveux humains, contenant dans un milieu approprié pour la teinture, au moins un précurseur de colorant d'oxydation de type ortho et/ou para, et au moins, à titre de coupleur, une métaphénylènediamine soufrée telle que définie dans les revendications 1, 3 ou 4.

8. Composition tinctoriale selon la revendication 7, caractérisée en ce qu'elle contient d'autres coupleurs choisis parmi les métadiphénols, des métaarninophénols, des métaphénylènediamines différentes de celles de formule (I), des métaacylaminophénols, des métauréidophénols, des métacarbalcoxyaminoph énols, l'α-naphtol, les dérivés indoliques, les coupleurs possédant un groupe méthylène actif.

9. Composition tinctoriale selon l'une quelconque des revendications 7 à 8, caractérisée en ce qu'elle contient 0, 05 à 3,5 % en poids du poids total de la composition d'au moins un composé de formule (I).

10. Composition tinctoriale selon l'une quelconque des revendications 7 à 9, caractérisée en ce qu'elle contient de 0,3 à 7 % en poids par rapport au poids total de la composition, de précurseurs de colorants d'oxydation de type ortho et/ou para et de coupleurs.

11. Composition tinctoriale selon l'une quelconque des revendications 7 à 10, caractérisée en ce qu'elle contient en outre un adjuvant choisi parmi des agents- tensio-actifs cationiques, anioniques, non-ioniques, amphotères ou leurs mélanges, en des concentrations comprises entre 0,5 % et 55 % en poids par rapport au poids total de la composition, les solvants organiques en des concentrations comprises entre 1 et 40 % en poids par rapport au poids total de la composition, les colorants directs, les agents épaississants en des concentrations comprises entre 0,1 et 5 % en poids par rapport au poids total de la composition et les agents antioxydants en des proportions comprises entre 0,05 et 1,5 % en poids par rapport au poids total de la composition.

12. Composition tinctoriale selon l'une quelconque des revendications 7 à 11, caractérisée en ce qu'elle se présente sous forme de liquide, de crème, de gel ou sous toute autre forme appropriée, ou peut être conditionnée en flacon aérosol en présence d'un agent propulseur et former des mousses.

13. Procédé de teinture d'oxydation des fibres kératiniques et en particulier des cheveux humains, caractérisé en ce que l'on applique sur les fibres kératiniques au moins un précurseur de colorant d'oxydation de type ortho et/ou para, et une métaphénylènediamine soufrée de formule générale : 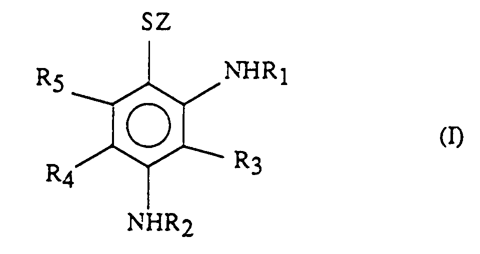 dans laquelle :
Z représente un radical alkyle en C₁-C₁₈, un radical aralkyle dans lequel le radical alkyle est en C₁-C₆, un radical monohydroxyalkyle en C₁-C₆ ou polyhydroxyalkyle C₂-C₆, un radical aryle, un radical fluoroalkyle en C₁-C₄, un radical aminoalkyle de formule : 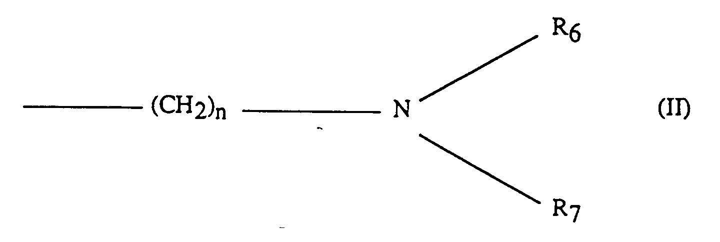 dans laquelle
n est un nombre entier compris entre 1 et 6 inclus, R₆ et R₇, identiques ou différents, représentent un atome d'hydrogène ou un radical alkyle en C₁-C₄, hydroxyalkyle en C₁-C₄, acyle en C₁-C₆;
R₁ et R₂, identiques ou différents, représentent un atome d'hydrogène, un radical alkyle en C₁-C₆, un radical monohydroxyalkyle en C₁-C₆, polyhydroxyalkyle en C₂-C₆, monocarbamylalkyle en C₁-C₆, dialkyl-carbamyle en C₁-C₆, aminoalkyle en C₁-C₆, acylaminoalkyle en C₁-C₄, carbalcoxy (C₂-C₆) alkyle (C₁-C₄), carbamyle ou monoalkyl en C₁-C₆ carbamyle, fluoroalkyle en C₁-C₄;
R₃ représente un atome d'hydrogène, un radical alkyle en C₁-C₄,
R₄ et R₅, identiques ou différents, représentent un atome d'hydrogène, un halogène, un radical alkyle en C₁-C₄, alcoxy en C₁-C₄, hydroxyalkyle en C₁-C₄, un radical SZ où Z à les significations indiquées ci-dessus, sous réserve qu'au moins un des radicaux R₃, R₄, R₅ soit différent d'un atome d'hydrogène et les sels d'acides correspondants, en présence d'un agent oxydant.

14. Procédé selon la revendication 13, caractérisé en ce que l'on mélange, au moment de l'emploi, une composition tinctoriale pour fibres kératiniques, et en particulier les cheveux humains, contenant, dans un milieu approprié pour la teinture, au moins un précurseur de colorant d'oxydation de type ortho et/ou para, et au moins, à titre de coupleur, une métaphénylènediamine soufrée telle que définie dans la revendication 13, avec une solution oxydante en une quantité suffisante pour développer la coloration, la composition résultante ayant un pH variant entre 2 et 13, et on applique le mélange ainsi obtenu sur les fibres kératiniques, et en particulier les cheveux humains.

15. Procédé de teinture d'oxydation selon l'une quelconque des revendications 13 à 14, caractérisé par le fait qu'on laisse poser pendant 10 à 40 minutes, de préférence 15 à 30 minutes, on rince les cheveux, on les lave au shampooing, on les rince à nouveau et on les sèche.

16. Nouvelles Métaphénylènediamines soufrées de formule générale : 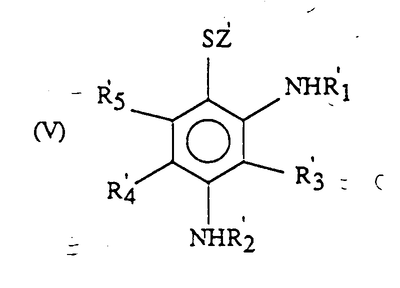 dans laquelle :
Z' représente un radical alkyle en C₁-C₁₈, un radical aralkyle dans lequel le radical alkyle est en C₁-C₆, un radical monohydroxyalkyle en C₁-C₆ ou polyhydroxyalkyle C₂-C₆, un radical aryle, un radical fluoroalkyle en C₁-C₄,un radical aminoalkyle de formule : 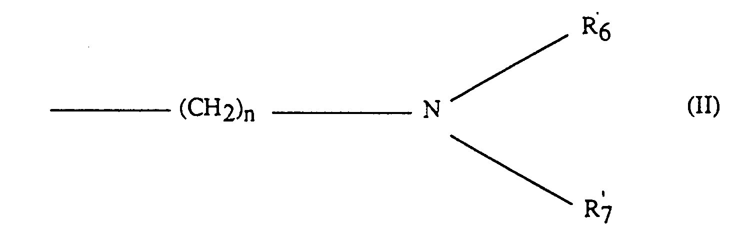 dans laquelle
n est un nombre entier compris entre 1 et 6 inclus; R'₆ et R'₇, identiques aux différents, représentent un atome d'hydrogène ou un radical alkyle en C₁-C₄, hydroxyalkyle en C₁-C₄, acyle en C₁-C₆;
R'₁ et R'₂, identiques ou différents, représentent un atome d'hydrogène, un radical alkyle en C₁-C₆, un radical monohydroxyalkyle en C₁-C₆, polyhydroxyalkyle en C₂-C₆, monocarbamylalkyle en C₁-C₆, dialkyl-carbamyle en C₁-C₆, aminoalkyle en C₁-C₆, acylaminoalkyle (C₁-C₄), carbalcoxy (C₂-C₆) alkyle (C₁-C₄), carbamyle ou monoalkyl en C₁-C₆ carbamyle, fluoroalkyle en C₁-C₄ ;
R'₃ représente un atome d'hydrogène, un radical alkyle en C₁-C₄;
R'4 et R'5, identiques ou différents, représentant un atome d'hydrogène, un halogène un radical alcoxy en C₁-C₄, hydroxyalkyle en C₁-C₄, un radical -SR avec R représentant un radical hydroxyalkyle en C₁-C₄, polyhydroxyalkyle en C₂-C₆, aralkyle dans lequel le radical alkyle est en C₁-C₆, un radical aryle, un radical aminoalkyle de formule : 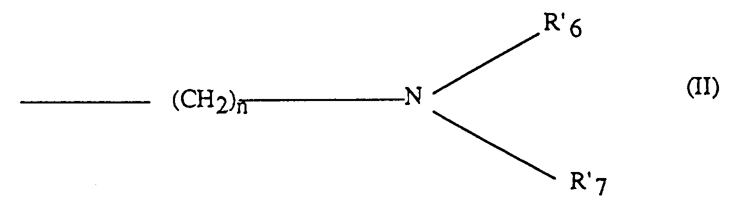 dans laquelle
n est un nombre entier compris entre 1 et 6 inclus; R'₆ et R'₇ identiques ou différents, représentent un atome d'hydrogène ou un radical alkyle en C₁-C₄, hydroxyalkyle en C₁-C₄ ou acyle en C₁-C₆, sous réserve que R'₄ et R'₅ ne désignent pas sirnultanément un atome d'hydrogène ;
et lorsque un des R'₄ ou R'₅ désigne un atome de chlore, R'₃ représente un radical alkyle.

17. Métaphénylènediamines soufrées selon la revendication 17, caractérisée en ce qu'elle est choisie parmi:
le 4-méthoxy 6-β-acétylaminoéthylthio 1,3-diaminobenzène
le 4-méthoxy 6-méthylthio 1,3-diaminobenzène
le 5-chloro 2-méthyl 4-β-acétylaminoéthylthio 1,3-diaminobenzène
le 4,6-bis-hydroxyéthyl thio 1,3-diaminobenzène

## Claims

1. Use of at least one sulphated metaphenylenediamine having general formula: 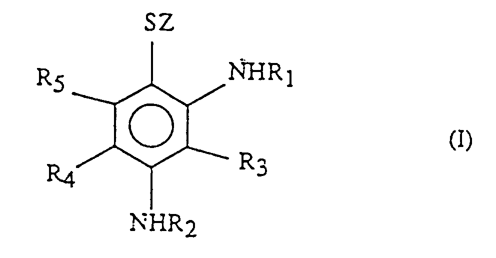 for dyeing keratinous fibres, in particular human hair, wherein:
Z represents a C₁-C₁₈ alkyl radical, an aralkyl radical wherein the alkyl radical is C₁-C₆, a C₁-C₆ monohydroxyalkyl or C₂-C₆ polyhydroxyalkyl radical, an aryl radical, a C₁-C₄ fluoroalkyl radical, an aminoalkyl radical having the formula: 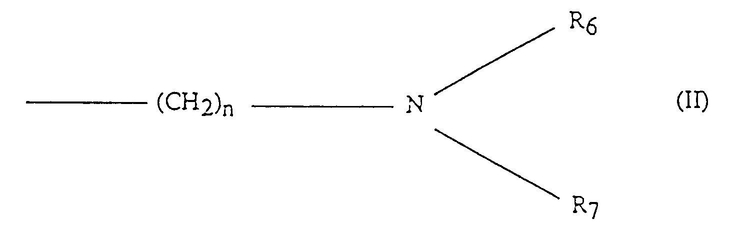 wherein
n is a whole number from 1 to 6 inclusive, R₆ and R₇, which may be identical or different, represent a hydrogen atom or a C₁-C₄ alkyl radical, a C₁-C₄ hydroxyalkyl radical or a C₁-C₆ acyl radical;
R₁ and R₂, which may be identical or different, represent a hydrogen atom, a C₁-C₆ alkyl radical, a C₁-C₆ monohydroxyalkyl radical, a C₂-C₆ polyhydroxyalkyl radical, a C₁-C₆ monocarbamylalkyl radical, a C₁-C₆ dialkylcarbamyl radical, a C₁-C₆ aminoalkyl radical, an acylaminoalkyl (C₁-C₄) radical, a carbalkoxy(C₂-C₆)alkyl(C₁-C₄) radical, a C₁-C₆ carbamyl or monoalkyl radical, or a C₁-C₄ fluoroalkyl radical;
R₃ represents a hydrogen atom or a C₁-C₄ alkyl radical;
R₄ and R₅, which may be identical or different, represent a hydrogen atom, a C₁-C₄ alkyl radical, a C₁-C₄ alkoxy radical, a C₁-C₄ hydroxyalkyl radical, a halogen, or a -SZ radical where Z has the meaning described above,
and at least one of the radicals R₃, R₄ or R₅ is other than a hydrogen atom,
and their corresponding acid salts.

2. Use according to claim 1 characterised in that the sulphated metaphenylenediamine having formula (I) is used in the presence of ortho and/or para type oxidation dye precursors.

3. Use according to claim 1 or claim 2 characterised in that in the compound having formula (I) Z represents a methyl, ethyl, propyl, butyl, dodecyl, hexadecyl, benzyl or phenyl radical, or a mono- or polyhydroxyalkyl radical selected from:
-CH₂-CH₂-OH, -CH₂-CHOH-CH₂-OH, -CH₂-CHOH-CH₃, or an aminoalkyl radical selected from:
-CH₂-CH₂-NH₂, -CH₂-CH₂-NHCH₃,
or an acylaminoalkyl radical selected from:
-CH₂-CH₂-NH-COCH₃ or 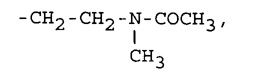 and when R₆ or R₇ designates an acyl radical, this represents a formyl, acetyl or propionyl group.

4. Use according to any one of claims 1 to 3 characterised in that the compounds having formula (I) are selected from the following:
2-methyl-4-methylthiol-1,3-diaminobenzene,
2-ethyl-4-methylthiol-1,3-diaminobenzene,
4-chloro-6-propylthiol-1,3-diaminobenzene,
4,6-bis-methylthio-1,3-diaminobenzene,
4,6-bis-ethylthiol-1,3-diaminobenzene,
4,6-bis-trifluoromethylthiol-1,3-[N,N'-bis-(2',2',2'trifluoroethyl)]-diaminobenzene,
4-methyl-6-methylthiol-1,3-diaminobenzene,
4-ethylthio-6-methyl-1,3-diaminobenzene,
4-carboxyethylthio-6-carboxymethylthiol-1,3-diaminobenzene,
4,6-bis-carboxyethylthiol-1,3-diaminobenzene,
2-methyl-4,6-bis-methylthio-1,3-diaminobenzene,
4,6-bis-ethylthio-2-methyl-1,3-diaminobenzene,
4,6-bis-propylthio-2-methyl-1,3-diaminobenzene,
4-methoxy-6-β-acetylaminoethylthio-1,3-diaminobenzene,
4-methoxy-6-methylthiol-1,3-diaminobenzene,
5-chloro-2-methyl-4-β-acetylaminoethylthio-1,3-diaminobenzene,
4,6-bis-hydroxyethylthio-1,3-diaminobenzene,
and their acid salts.

5. Use according to any one of claims 1 to 4 characterised in that the corresponding acid salts are selected from hydrochlorides, sulphates or hydrobromides.

6. Use according to any one of claims 2 to 5 characterised in that the oxidation dye precursors are selected from paraphenylenediamines, paraaminophenols, para heterocyclic precursors derived from pyridine, pyrimidine or pyrazole, orthoaminophenols and bis-phenylalkylenediamines.

7. A dye composition for keratinous fibres, particularly human hair, containing at least one ortho and/or para type oxidation dye precursor and at least one sulphated metaphenylenediamine as defined in claim 1, 3 or 4 as a coupling agent, in an appropriate dye medium.

8. Dye composition according to claim 7 characterised in that it contains other coupling agents selected from metadiphenols, metaaminophenols, metaphenylenediamines other than those of formula (I), metaacylaminophenols, metaureidophenols, metacarbalkoxyaminophenols, α-napthol, indole derivatives or coupling agents having an active methylene group.

9. Dye composition according to claim 7 or claim 8 characterised in that it contains 0.05 to 3.5% by weight with respect to the total composition weight of the compound having formula (I).

10. Dye composition according to any one of claims 7 to 9 characterised in that it contains 0.3 to 7% by weight with respect to the total composition weight of ortho and/or para type oxidation dye precursors and coupling agents.

11. Dye composition according to any one of claims 7 to 10 characterised in that it further contains an additive selected from: a cationic, anionic, non-ionic or amphoteric surfactant or mixture thereof in concentrations of between 0.5 and 55% by weight with respect to the total composition weight, organic solvents in concentrations of between 1 and 40% by weight with respect to the total composition weight, direct dyes, thickening agents in concentrations of between 0.1 and 5% by weight with respect to the total composition weight and antioxidants in proportions of between 0.05 and 1.5% by weight with respect to the total composition weight.

12. Dye composition according to any one of claims 7 to 11 characterised in that it is in the form of a liquid, cream, gel or any other appropriate form or packaged in an aerosol can in the presence of a propellant and adapted to form a foam.

13. A method of dyeing keratinous fibres, in particular human hair, wherein at least one ortho and/or para type oxidation dye precursor is applied to the keratinous fibres with a sulphated metaphenylenediamine having the general formula: 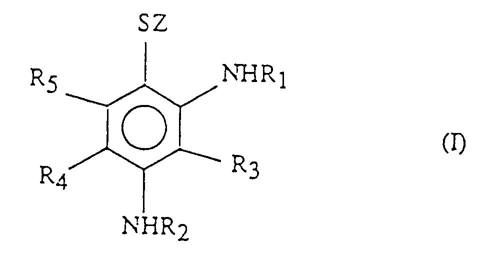 wherein
Z represents a C₁-C₁₈ alkyl radical, an aralkyl radical wherein the alkyl radical is C₁-C₆, a C₁-C₆ monohydroxyalkyl or C₂-C₆ polyhydroxyalkyl radical, an aryl radical, a C₁-C₄ fluoroalkyl radical, an aminoalkyl radical having the formula: 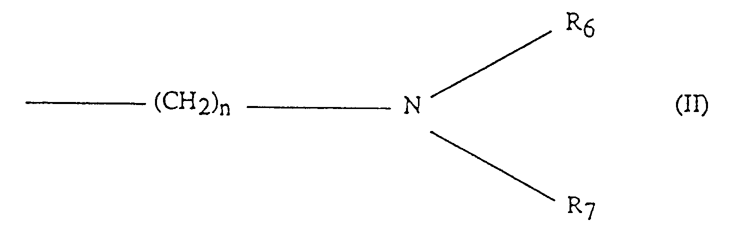 wherein
n is a whole number from 1 to 6 inclusive, R₆ and R₇, which may be identical or different, represent a hydrogen atom or a C₁-C₄ alkyl radical, a C₁-C₄ hydroxyalkyl radical or a C₁-C₆ acyl radical;
R₁ and R₂, which may be identical or different, represent a hydrogen atom, a C₁-C₆ alkyl radical, a C₁-C₆ monohydroxyalkyl radical, a C₂-C₆ polyhydroxyalkyl radical, a C₁-C₆ monocarbamylalkyl radical, a C₁-C₆ dialkylcarbamyl radical, a C₁-C₆ aminoalkyl radical, an acylaminoalkyl (C₁-C₄) radical, a carbalkoxy(C₂-C₆)alkyl(C₁-C₄) radical, a C₁-C₆ carbamyl or monoalkyl radical, or a C₁-C₄ fluoroalkyl radical;
R₃ represents a hydrogen atom or a C₁-C₄ alkyl radical;
R₄ and R₅, which may be identical or different, represent a hydrogen atom, a halogen, a C₁-C₄ alkyl radical, a C₁-C₄ alkoxy radical, a C₁-C₄ hydroxyalkyl radical or a SZ radical where Z has the meaning described above,
provided that at least one of the radicals R₃, R₄ or R₅ is other than a hydrogen atom,
and their corresponding acid salts,
in the presence of an oxidising agent.

14. Method according to claim 13 characterised in that a dye composition for keratinous fibres, in particular human hair, containing at least one ortho and/or para type oxidation dye precursor and at least one sulphated metaphenylenediamine as defined in claim 13 in an appropriate medium is mixed just before use with a sufficient quantity of an oxidising solution to develop the dye, the resultant composition having a pH of between 2 and 13 and being applied to the keratinous fibres.

15. Oxidation dyeing method according to claim 13 or claim 14 characterised in that said composition is left on the hair for 10 to 40 minutes, preferably 15 to 30 minutes, the hair is rinsed, shampooed, rinsed again and dried.

16. Novel sulphated metaphenylenediamines having the general formula: 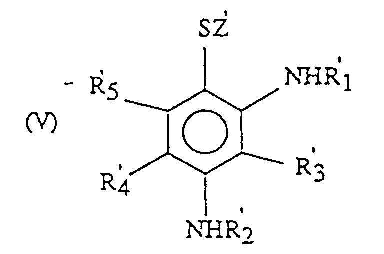 wherein:
Z' represents a C₁-C₁₈ alkyl radical, an aralkyl radical wherein the alkyl radical is C₁-C₆, a C₁-C₆ monohydroxyalkyl or C₂-C₆ polyhydroxyalkyl radical, an aryl radical, a C₁-C₄ fluoroalkyl radical, an aminoalkyl radical having the formula: 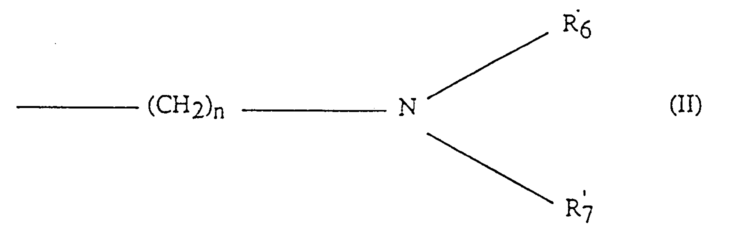 wherein
n is a whole number from 1 to 6 inclusive; R'₆ and R'₇, which may be identical or different, represent a hydrogen atom or a C₁-C₄ alkyl radical, a C₁-C₄ hydroxyalkyl radical or a C₁-C₆ acyl radical;
R'₁ and R'₂, which may be identical or different, represent a hydrogen atom, a C₁-C₆ alkyl radical, a C₁-C₆ monohydroxyalkyl radical, a C₂-C₆ polyhydroxyalkyl radical, a C₁-C₆ monocarbamylalkyl radical, a C₁-C₆ dialkylcarbamyl radical, a C₁-C₆ aminoalkyl radical, an acylaminoalkyl (C₁-C₄) radical, a carbalkoxy(C₂-C₆)alkyl(C₁-C₄) radical, a C₁-C₆ carbamyl or monoalkyl radical, or a C₁-C₄ fluoroalkyl radical;
R'₃ represents a hydrogen atom or a C₁-C₄ alkyl radical;
R'₄ and R'₅, which may be identical or different, represent a hydrogen atom, a halogen, a C₁-C₄ alkoxy radical, a C₁-C₄ hydroxyalkyl radical, a -SR radical where R represents a C₁-C₄ hydroxyalkyl radical, a C₂-C₆ polyhydroxyalkyl radical, an aralkyl radical wherein the alkyl radical is C₁-C₆, an aryl radical, an aminoalkyl radical having formula: 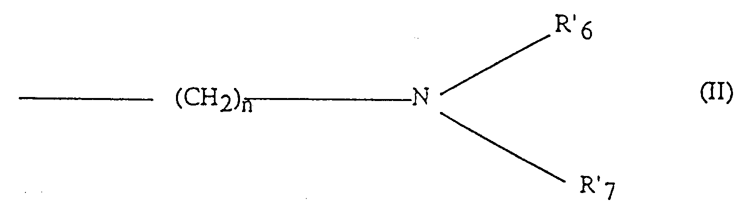 wherein
n is a whole number from 1 to 6 inclusive;
R'₆ and R'₇, which may be identical or different, represent a hydrogen atom or a C₁-C₄ alkyl radical, a C₁-C₄ hydroxyalkyl radical or a C₁-C₆ acyl radical, providing that R'₄ and R'₅ do not simultaneously designate a hydrogen atom;
and when either R'₄ or R'₅ designates a chlorine atom, R'₃ represents an alkyl radical.

17. Sulphated metaphenylenediamines according to claim 17 characterised in that they are selected from:
4-methoxy-6-β-acetylaminoethylthio-1,3-diaminobenzene,
4-methoxy-6-methylthiol-1,3-diaminobenzene,
5-chloro-2-methyl-4-β-acetylaminoethylthio-1,3-diaminobenzene,
4,6-bis-hydroxyethylthio-1,3-diaminobenzene.

## Patentansprüche

1. Zur Färbung keratinischer Fasern und insbesondere der menschlichen Haare vorgesehene Verwendung mindestens eines schwefelhaltigen m-Phenylendiamins der allgemeinen Formel: 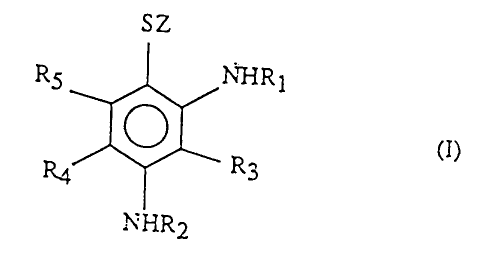 worin gilt:
Z stellt einen C₁₋₁₈-Alkyl-, einen Aralkylrest, worin der Alkylrest ein C₁₋₆-Rest ist, einen C₁₋₆-Monohydroxyalkyl- oder C₂-₆-Polyhydroxyalkyl-, Aryl-, C₁-₄-Fluoralkyl- oder einen Aminoalkylrest der Formel dar: 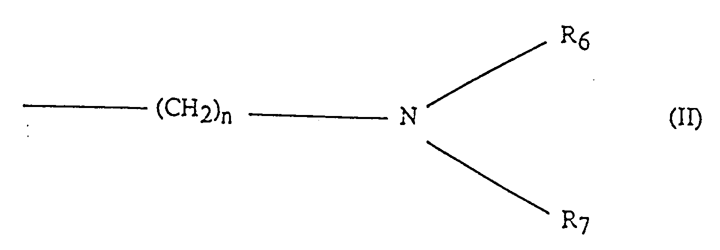 worin
n eine ganze Zahl von 1 bis 6 ist, und worin R₆ und R₇, gleich oder verschieden, ein Wasserstoffatom oder einen C₁₋₄-Alkyl-, C₁₋₄-Hydroxyalkyl- und einen C₁₋₆-Acylrest darstellen;
R₁ und R₂ stellen, gleich oder verschieden, ein Wasserstoffatom, einen C₁₋₆-Alkyl-, C₁₋₆-Monohydroxyalkyl-, C₂₋₆-Polyhydroxyalkyl-, C₁₋₆-Monocarbamylalkyl, C₁₋₆-Dialkylcarbamyl, C₁₋₆-Aminoalkyl-, C₁₋₄-Acylaminoalkyl-, C₂₋₆-Carbalkoxy-C₁₋₄-alkyl-, einen Carbamyl- oder C₁₋₆-Monoalkylcarbamyl und einen C₁₋₄-Fluoralkylrest dar;
R₃ stellt ein Wasserstoffatom oder einen C₁₋₄-Alkylrest dar;
R₄ und R₅ stellen, gleich oder verschieden, ein Wasserstoffatom, einen C₁₋₄-Alkyl-, C₁₋₄-Hydroxyalkylrest, ein Halogenatom und einen Rest -SZ dar, worin Z die oben angegebenen Bedeutungen hat,
mit der Maßgabe, daß mindestens einer der Reste R₃, R₄ oder R₅ sich von einem Wasserstoffatom unterscheidet,
sowie von deren entsprechenden Säuresalzen.

2. Verwendung gemäß Anspruch 1,
dadurch **gekennzeichnet**, daß
das schwefelhaltige m-Phenylendiamin der Formel (I) in Gegenwart von Oxidationsfarbstoff-Vorstufenverbindungen vom ortho- und/oder para-Typ angewandt wird.

3. Verwendung gemäß Anspruch 1 oder 2,
dadurch **gekennzeichnet**, daß
in der Verbindung der Formel (I) Z einen Methyl-, Ethyl-, Propyl-, Butyl-, Dodecyl-, Hexadecylrest, den Benzylrest, den Phenylrest, einen Mono- oder Polyhydroxyalkylrest, ausgewählt aus -CH₂-CH₂OH, -CH₂-CHOH-CH₂-OH oder -CH₂-CHOH-CH₃,
einen Aminoalkylrest, ausgewählt aus: -CH₂-CH₂-NH₂ oder -CH₂-CH₂-NHCH₃,
einen Acylaminoalkylrest bedeutet, ausgewählt aus -CH₂-CH₂-NHCOCH₃ oder -CH₂-CH₂-N(CH₃)COCH₃),
und ferner gilt:
wenn R₆ und R₇ einen Acylrest bedeuten, stellt dieser vorzugsweise eine Formyl-, Acetyl- oder Propionylgruppe dar.

4. Verwendung gemäß einem der Ansprüche 1 bis 3,
dadurch **gekennzeichnet**, daß
die Verbindungen der Formel (I) aus den folgenden Verbindungen ausgewählt sind:
2-Methyl-4-methylthiol-1,3-diaminobenzol
2-Ethyl-4-methylthio-1,3-diaminobenzol
4-Chlor-6-propylthio-1,3-diaminobenzol
4,6-Bismethylthio-1,3-diaminobenzol
4,6-Bisethylthio-1,3-diaminobenzol
4,6-Bistrifluormethylthio-1,3-[N,N'-bis(2',2',2'-trifluorethyl)]diaminobenzol
4-Methyl-6-methylthio-1,3-diaminobenzol
4-Ethylthio-6-methyl-1,3-diaminobenzol
4-Carboxyethylthio-6-carboxymethylthio-1,3-diaminobenzol
4,6-Biscarboxyethylthio-1,3-diaminobenzol
2-Methyl-4,6-bismethylthio-1,3-diaminobenzol
4,6-Bisethylthio-2-methyl-1,3-diaminobenzol
4,6-Bispropylthio-2-methyl-1,3-diaminobenzol
4-Methoxy-6-β-acetylaminoethylthio-1,3-diaminobenzol
4-Methoxy-6-methylthio-1,3-diaminobenzol
5-Chlor-2-methyl-4-β-acetylaminoethylthio-1,3-diaminobenzol
4,6-Bishydroxyethylthio-1,3-diaminobenzol
und aus deren Säuresalzen.

5. Verwendung gemäß einem der Ansprüche 1 bis 4,
dadurch **gekennzeichnet**, daß
die entsprechenden Säuresalze aus Hydrochloriden, Sulfaten oder Hydrobromiden ausgewählt sind.

6. Verwendung gemäß einem der Ansprüche 2 bis 5,
dadurch **gekennzeichnet**, daß
die Oxidationsfarbstoff-Vorstufenverbindungen aus p-Phenylendiaminen, p-Aminophenolen, para-Derivaten heterozyklischer Vorstufenverbindungen des Pyridins, Pyrimidins oder Pyrazols, aus o-Aminophenolen und Bisphenylalkylendiaminen ausgewählt sind.

7. Färbezusammensetzung für keratinische Fasern und insbesondere für die menschlichen Haare, enthaltend in einem zur Färbung geeigneten Milieu mindestens eine Oxidationsfarbstoff-Vorstufenverbindung vom ortho- oder para-Typ und mindestens, als Kuppler, ein in einem der Ansprüche 1, 3 oder 4 definiertes schwefelhaltiges m-Phenylendiamin.

8. Färbezusammensetzung gemäß Anspruch 7,
dadurch **gekennzeichnet**, daß
sie weitere Kuppler enthält, ausgewählt aus m-Diphenolen, m-Aminophenolen, m-Phenylendiaminen, die sich von denen der Formel (I) unterscheiden, aus m-Acylaminophenolen, m-Ureidophenolen, m-Carbalkoxyaminophenolen, α-Naphthol, Indolderivaten und aus Kupplern mit einer aktiven Methylengruppe.

9. Färbezusammensetzung gemäß einem der Ansprüche 7 und 8,
dadurch **gekennzeichnet**, daß
sie 0,05 bis 3,5 Gew.%, bezogen auf das Gesamtgewicht der Zusammensetzung, mindestens einer Verbindung der Formel (I) enthält.

10. Färbezusammensetzung gemäß einem der Ansprüche 7 bis 9,
dadurch **gekennzeichnet**, daß
sie 0,3 bis 7 Gew.%, bezogen auf das Gesamtgewicht der Zusammensetzung, OXidationsfarbstoff-Vorstufenverbindungen vom ortho- oder para-Typ und Kuppler enthält.

11. Färbezusammensetzung gemäß einem der Ansprüche 7 bis 10,
dadurch **gekennzeichnet**, daß
sie ausserdem einen Hilfsstoff, ausgewählt aus oberflächenaktiven kationischen, anionischen, nicht-ionischen und amphoteren Mitteln oder aus deren Mischungen, in Konzentrationen von 0,5 bis 55 Gew.%, bezogen auf das Gesamtgewicht der Zusammensetzung, organische Lösungsmittel in Konzentrationen von 1 bis 40 Gew.%, bezogen auf das Gesamtgewicht der Zusammensetzung, Direktfarbstoffe, Verdickungsmittel in Konzentrationen von 0,1 bis 5 Gew.%, bezogen auf das Gesamtgewicht der Zusammensetzung, und Antioxidantien in Mengenanteilen von 0,05 bis 1,5 Gew.%, bezogen auf das Gesamtgewicht der Zusammensetzung, enthält.

12. Färbezusammensetzung gemäß einem der Ansprüche 7 bis 11,
dadurch **gekennzeichnet**, daß
sie in Form einer Flüssigkeit, Creme, eines Gels oder in jeder weiteren geeigneten Form vorliegt oder als Aerosol-Fläschchen in Gegenwart eines Treibmittels zubereitet ist und Schäume bildet.

13. Verfahren zur Oxidationsfärbung keratinischer Fasern und insbesondere der menschlichen Haare,
dadurch **gekennzeichnet,** daß
man auf die keratinischen Fasern mindestens eine Oxidationsfarbstoff-Vorstufenverbindung vom ortho- und/oder para-Typ und ein schwefelhaltiges m-Phenylendiamin der allgemeinen Formel: 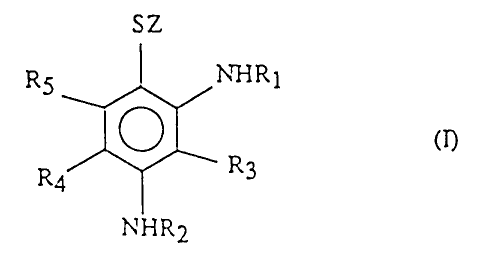 worin gilt:
Z stellt einen C₁₋₁₈-Alkyl-, einen Aralkylrest, worin der Alkylrest ein C₁₋₆-Rest ist, einen C₁₋₆-Monohydroxyalkyl- oder C₂₋₆-Polyhydroxyalkyl, Aryl-, C₁₋₄-Fluoralkyl- oder einen Aminoalkylrest der Formel dar: 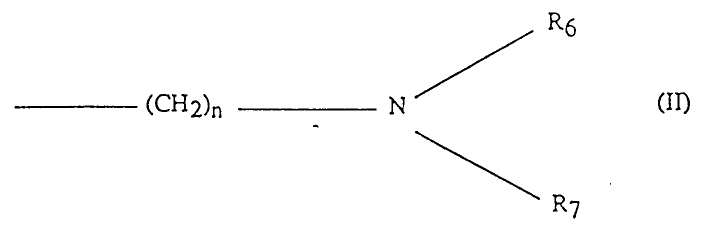 worin
n eine ganze Zahl von 1 bis 6 ist, und worin R₆ und R₇, gleich oder verschieden, ein Wasserstoffatom oder einen C₁₋₄-Alkyl-, C₁₋₄-Hydroxyalkyl- und einen C₁₋₆-Acylrest darstellen;
R₁ und R₂ stellen, gleich oder verschieden, ein Wasserstoffatom, einen C₁₋₆-Alkyl-, C₁₋₆-Monohydroxyalkyl-, C₂₋₆-Polyhydroxyalkyl-, C₁₋₆-Monocarbamylalkyl-, C₁₋₆-Dialkylcarbamyl, C₁₋₆-Aminoalkyl-, C₁₋₄-Acylaminoalkyl-, C₂₋₆-Carbalkoxy-C₁₋₄-alkyl-, einen Carbamyl- oder C₁₋₆-Monoalkylcarbamyl- und einen C₁₋₄-Fluoralkylrest dar;
R₃ stellt ein Wasserstoffatom oder einen C₁₋₄-Alkylrest dar;
R₄ und R₅ stellen, gleich oder verschieden, ein Wasserstoffatom, einen C₁₋₄-Alkyl-, C₁₋₄-Hydroxyalkylrest, ein Halogenatom und einen Rest -SZ dar, worin Z die oben angegebenen Bedeutungen hat,
mit der Maßgabe, daß mindestens einer der Reste R₃, R₄ oder R₅ sich von einem Wasserstoffatom unterscheidet,
sowie die entsprechenden Säuresalze in Gegenwart eines oxidierenden Mittels aufbringt.

14. Verfahren gemäß Anspruch 13,
dadurch **gekennzeichnet**, daß
man, zum Zeitpunkt der Anwendung, eine Färbezusammensetzung für keratinische Fasern und insbesondere für die menschlichen Haare, die, in einem zur Färbung geeigneten Milieu, mindestens eine Oxidationsfarbstoff-Vorstufenverbindung vom ortho- und/oder para-Typ und mindestens, als Kuppler, ein im Anspruch 13 definiertes schwefelhaltiges m-Phenylendiamin enthält, mit einer oxidierenden Lösung in einer zur Entwicklung der Färbung ausreichenden Menge vermischt, wobei die sich ergebende Zusammensetzung einen pH-Wert von 2 bis 13 aufweist, und daß man die so erhaltene Mischung auf die keratinischen Fasern und insbesondere die menschlichen Haare aufbringt.

15. Verfahren zur Oxidationsfärbung gemäß einem der Ansprüche 13 oder 14,
dadurch **gekennzeichnet**, daß
man die Mischung 10 bis 40 und vorzugsweise 15 bis 30 Minuten lang verweilen läßt, die Haare spült, sie unter Schamponieren wäscht, sie erneut spült und dann trocknet.

16. Neue schwefelhaltige m-Phenylendiamine der allgemeinen Formel: 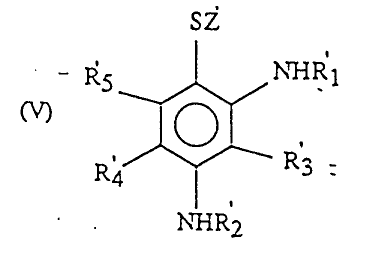 worin gilt:
Z' stellt einen C₁₋₁₈-Alkyl-, einen Aralkylrest, worin der Alkylrest ein C₁₋₆-Rest ist, einen C₁₋₆-Monohydroxyalkyl- oder C₂₋₆-Polyhydroxyalkyl-, Aryl-, C₁₋₄-Fluoralkyl- oder einen Aminoalkylrest der Formel dar: 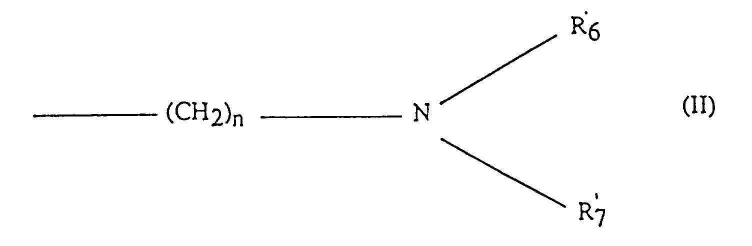 worin
n eine ganze Zahl von 1 bis 6 ist, und
worin
R'₆ und R'₇, gleich oder verschieden, ein Wasserstoffatom oder einen C₁₋₄-Alkyl-, C₁₋₄-Hydroxyalkyl- und einen C₁₋₆-Acylrest darstellen;
R'₁ und R'₂ stellen, gleich oder verschieden, ein Wasserstoffatom, einen C₁₋₆-Alkyl-, C₁₋₆-Monohydroxyalkyl-, C₂₋₆-Polyhydroxyalkyl, C₁₋₆-Monocarbamylalkyl-, C₁₋₆-Dialkylcarbamyl-, C₁₋₆Aminoalkyl-, C₁₋₄-Acylaminoalkyl-, C₂₋₆-Carbalkoxy-C₁₋₄-alkyl-, einen Carbamyl- oder C₁₋₆-Monoalkylcarbamyl und einen C₁₋₄-Fluoralkylrest dar;
R'₃ stellt ein Wasserstoffatom oder einen C₁₋₄-Alkylrest dar;
R'₄ und R'₅ stellen, gleich oder verschieden, ein Wasserstoff-, Halogenatom, einen C₁₋₄-Alkoxy-, C₁₋₄-Hydroxyalkylrest und einen Rest -SR dar, worin R einen C₁₋₄-Hydroxyalkyl-, C₂₋₆-Polyhydroxyalkyl-, Aralkylrest, worin der Alkylrest ein C₁₋₆-Rest ist, einen Aryl- und Aminoalkylrest der Formel dar: 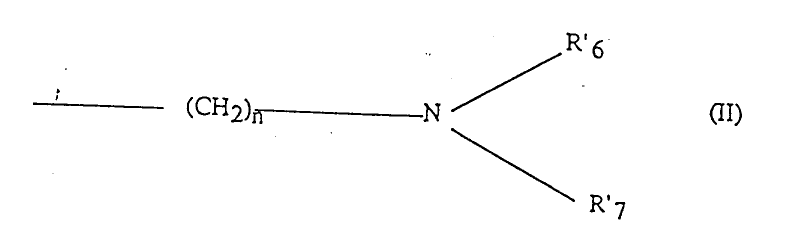 worin
n eine ganze Zahl von 1 bis 6 ist, und worin R'₆ und R'₇, gleich oder verschieden, ein Wasserstoffatom oder einen C₁₋₄-Alkyl-, C₁₋₄-Hydroxyalkyl- oder einen C₁₋₆-Acylrest darstellen,
mit der Maßgabe, daß R'₄ und R'₅ nicht gleichzeitig ein Wasserstoffatom bedeuten;
und wenn einer der Reste R'₄ oder R'₅ ein Chloratom bedeutet, stellt R'₃ einen Alkylrest dar.

17. Schwefelhaltige m-Phenylendiamine gemäß Anspruch 16,
dadurch **gekennzeichnet**, daß
sie ausgewählt sind aus:
4-Methoxy-6-β-acetylaminoethylthio-1,3-diaminobenzol,
4-Methoxy-6-methylthio-1,3-diaminobenzol,
5-Chlor-2-methyl-4-β-acetylaminoethylthio-1,3-diaminobenzol
und aus 4,6-Bishydroxyethylthio-1,3-diaminobenzol.
